# EUROPEAN PATENT SPECIFICATION

(11) **EP 2 042 189 B1**
(45) Date of publication and mention of the grant of the patent: **26.06.2013**
(21) Application number: 07765882.1
(22) Date of filing: 21.05.2007
(51) Int. Cl.: A61K 38/28, A61P 25/28

(54) **USE OF PROINSULIN FOR THE PREPARATION OF A NEUROPROTECTIVE PHARMACEUTICAL COMPOSITION, THERAPEUTIC COMPOSITION CONTAINING IT AND APPLICATIONS THEREOF**
VERWENDUNG VON PROINSULIN ZUR HERSTELLUNG EINER NEUROPROTEKTIVEN PHARMAZEUTISCHEN ZUSAMMENSETZUNG, DIESE ENTHALTENDE THERAPEUTISCHE ZUSAMMENSETZUNG UND IHRE ANWENDUNG
UTILISATION DE LA PROINSULINE POUR L'ÉLABORATION D'UNE COMPOSITION PHARMACEUTIQUE NEUROPROTECTRICE, COMPOSITION THÉRAPEUTIQUE CONTENANT CELLE-CI ET SES APPLICATIONS

(30) Priority: 22.05.2006 ES 200601314
(43) Date of publication of application: 01.04.2009
(73) Proprietor: Consejo Superior de Investigaciones Cientificas (CSIC), 28006 Madrid (ES); Universidad De Alcalá De Henares, 28801 Alcalá De Henares (ES); UNIVERSIDAD AUTONOMA DE BARCELONA-UAB, E-08193 Bellaterra, Barcelona (ES)
(72) Inventor: DE LA ROSA CANO, Enrique J., E-28040 Madrid (ES); DE PABLO DÁVILA, Maria Flora, E-28040 Madrid (ES); BOYA TREMOLEDA, Patricia, E-28040 Madrid (ES); CORROCHANO SÁNCHEZ, Silvia, E-28040 Madrid (ES); DE LA VILLA POLO, Pedro, E-28871 Alcalá De Henares (ES); BARHOUM TANNOUS, Rima, E-28871 Alcalá De Henares (ES); BOSCH TUBERT, Fátima, E-08193 Bellaterra (ES)
(74) Representative: ABG Patentes, S.L.
(86) International application number: PCT/ES2007/070097
(87) International publication number: WO 2007/135220

(56) References cited:
- GB-A- 2 104 380
- CORROCHANO SANCHEZ S ET AL: "Insulina an muerte celular programada: expresion and efectos durante la neurogenesis in la retina de raton" CONGRESO DE LA SOCIEDAD ESPANOLA DE BIOLOGIA CELULAR,, 3 November 2005 (2005-11-03), page 90, XP008092512
- WATSON G STENNIS ET AL: "Preserved cognition in patients with early Alzheimer disease and amnestic mild cognitive impairment during treatment with rosiglitazone: a preliminarystudy" AMERICAN JOURNAL OF GERIATRIC PSYCHIATRY, AMERICAN PSYCHIATRY PRESS, WASHINGTON, DC, US, vol. 13, no. 11, 1 November 2005 (2005-11-01), pages 950-958, XP009090008 ISSN: 1064-7481
- CRAFT S ET AL: "Memory improvement following induced hyperinsulinemia in Alzheimer's disease." NEUROBIOLOGY OF AGING 1996 JAN-FEB, vol. 17, no. 1, January 1996 (1996-01), pages 123-130, XP002561902 ISSN: 0197-4580
- HERNÁNDEZ-SÁNCHEZ C ET AL: "Proinsulin in development: new roles for an ancient prohormone" DIABETOLOGIA ; CLINICAL AND EXPERIMENTAL DIABETES AND METABOLISM, SPRINGER, BERLIN, DE, vol. 49, no. 6, 5 April 2006 (2006-04-05), pages 1142-1150, XP019417798 ISSN: 1432-0428
- DIAZ B. ET AL.: 'Apoptotic cell death of proliferating neuroepithelial cells in the embryonic retina is prevented by insulin' EUROPEAN JOURNAL OF NEUROSCIENCE vol. 11, no. 5, May 1999, pages 1624 - 1632, XP008090592
- DIAZ B. ET AL.: 'In vivo regulation of death by embryonic (pro)insulin and the insulin receptor during early retinal neurogenesis' DEVELOPMENT vol. 127, no. 8, 15 April 2000, pages 1641 - 1649, XP008090540
- CORROCHANO SANCHEZ S. ET AL.: 'Insulina an muerte celular programada: expresion and efectos durante la neurogenesis in la retina de raton' XI CONGRESO OF THE SOCIEDAD SPANISH DE BIOLOGIA CELULAR. CADIZ 03 November 2005 - 06 November 2005, page 90, XP008092512
- VALENCIANO A.I. ET AL.: 'Proinsulin/insulin is synthesized locally and prevents caspase- and cathepsin-mediated cell death in the embryonic mouse retina' JOURNAL OF NEUROCEMISTRY vol. 99, no. 2, 01 October 2006, pages 524 - 536, XP008092214
- SCHLICHTENBREDE F C ET AL: "Intraocular gene delivery of ciliary neurotrophic factor results in significant loss of retinal function in normal mice and in the Prph2Rd2/Rd2 model of retinal degeneration.", GENE THERAPY MAR 2003 LNKD- PUBMED:12621456, vol. 10, no. 6, March 2003 (2003-03), pages 523-527, ISSN: 0969-7128
- OTANI ATSUSHI ET AL: "Rescue of retinal degeneration by intravitreally injected adult bone marrow-derived lineage-negative hematopoietic stem cells.", THE JOURNAL OF CLINICAL INVESTIGATION SEP 2004 LNKD- PUBMED:15372100, vol. 114, no. 6, September 2004 (2004-09), pages 765-774, ISSN: 0021-9738
- BOWES C ET AL: "Retinal degeneration in the rd mouse is caused by a defect in the beta subunit of rod cGMP-phosphodiesterase.", NATURE 18 OCT 1990 LNKD- PUBMED:1977087, vol. 347, no. 6294, 18 October 1990 (1990-10-18), pages 677-680, ISSN: 0028-0836

## Description

### Field of the Invention

The present invention is comprised within the biomedicine field and more specifically within the development of therapeutic compounds. The invention particularly relates to the specific use of the proinsulin molecule for the treatment of retinal degenerative diseases such as retinitis pigmentosa, as well as other neurodegenerative conditions.

### State of the Art

Neurodegenerative diseases comprise a variety of progressive disorders of the central nervous system with a genetic-hereditary, traumatic, sporadic or senile origin. Most neurodegenerative diseases present programmed cell death of neurons and/or glial cells in their origin or their progression. Said death process, forming an irreversible step of the damage to the nervous tissue, appears independently of the primary cause of the disorder, be it genetic, traumatic, sporadic or senile.

A number of growth factors have an essential role in the regulation of the balance between the life and death of different cell types, including neurons and glial cells. These include the members of the insulin family including insulin, its precursor proinsulin, and IGF-I and IGF-II (Varela-Nieto, I., de la Rosa, E.J., Valenciano, A.I., and Leon, Y. (2003) Cell death in the nervous system: lessons from insulin and insulin-like growth factors. Mol Neurobiol 28: 23-50). The retina forms part of the central nervous system and is a well established model for studying both physiological and pathological processes of the nervous system; for this reason, it is the cell model used in the present invention. One of the most important pathological processes studied the retina is the so-called retinitis pigmentosa, because this pathology comprises a wide group of hereditary retinal disorders and represents one of the greatest causes of blindness in the world, with an approximate incidence of one in 4,000 persons. Although more than 120 involved loci have been characterized and there are different etiologies, in all cases there is a chronic and progressive loss due to programmed cell death of the retinal neurons, specifically of the photoreceptors, making the individuals blind. There is currently no treatment for retinitis pigmentosa and for the time being, neuroprotective, gene therapy, neurorepairing and bioengineering strategies are only being undertaken in animal models with degeneration, such as rd mice, RCS rats and other models in dogs, cats, pigs and even Drosophila. The rd1 (rod degeneration) mouse was one of the first models for studying molecular and cell mechanisms determining cell degeneration, the apoptotic nature of photoreceptors death having been determined (Chang, G.Q., Hao, Y., and Wong, F. (1993) Apoptosis: final common pathway of photoreceptor death in rd, rds, and rhodopsin mutant mice. 30 Neuron 11: 595-605). The different rd mice provide an ideal model for the assay of new therapeutic approaches to the treatment of hereditary retinal dystrophies, because they allow studying the degenerative process of photoreceptors from a molecular, cellular and genetic point of view.

Gene therapy interventions tend to reintroduce a functional copy of the mutated gene causing neurodegeneration. Progress has been made with recombinant adenoviruses or viral vectors associated to adenoviruses. Specifically, the replacement of the β subunit of rod-specific cGMP phosphodiesterase (βPDE) in newborn rd mice, achieving a histological rescue of rd phenotype of at least 6 weeks (Bennett, J., Tanabe, T., Sun, D., Zeng, Y., Kjeldbye, H., Gouras, P., and Maguire, A.M. (1996) Photoreceptor cell rescue in retinal degeneration (rd) mice by in vivo gene therapy. Nat Med 2: 649-654). However, this therapy requires the unequivocal identification of the mutated gene in each patient, which is currently only possible in 40% of cases (Wang, D.Y., Chan, W.M., Tam, P.O., Baum, L., Lam, D.S., Chong, K.K., Fan, B.J., and Pang, C.P. (2005) Gene mutations in retinitis pigmentosa and their clinical implications. Clin Chim Acta 351: 5-16).

The transplant of neural stem cells or precursors for the purpose of developing new photoreceptors is the purpose of neurorepairing therapies. New photoreceptors have to reestablish the suitable connections with the neurons of the internal retina.

Neuroprotection that is induced by means of treatment with growth factors seeks to prevent cell death associated to the neurodegenerative process. Different forms of administration have been tested in several animal models with retinal degeneration. The first attempts consisted of intravitreal injections of several recombinant proteins in rats or mice with retinal degeneration (Faktorovich, E.G., Steinberg, R.H., Yasumura, D., Matthes, M.T., and LaVail, M.M. (1990) Photoreceptor degeneration in inherited retinal dystrophy delayed by basic fibroblast growth factor. Nature 347: 83-86; LaVail, M.M., Unoki, K., Yasumura, D., Matthes, M.T., Yancopoulos, G.D., and Steinberg, R.H. (1992) Multiple growth factors, cytokines, and neurotrophins rescue photoreceptors from the damaging effects of constant light. Proc Natl Acad Sci USA 89: 11249-11253). These experiments demonstrated that FGF2 slowed down photoreceptor degeneration in RCS rats (Royal Collage of Surgeon). Several survival factors, including FGF2, FGF1, BDNF and CNTF, decreased photoreceptor death induced by light damage (LaVail, M.M., Yasumura, D., Matthes, M.T., Lau-Villacorta, C., Unoki, K., Sung, C.H., and Steinberg, R.H. 15 (1998) Protection of mouse photoreceptors by survival factors in retinal degenerations. Invest Ophthalmol Vis Sci 39: 592-602). Intravitreal injections of CNTF analogs in mouse models with hereditary retinal degenerations (model Q433ter, rd and nr; terminology used to designate certain mouse models with retinitis pigmentosa) gave rise to an evident improvement of some degenerations. The neuroprotective effect due to CNTF analogs has also been verified in studies in cats with autosomal dominant cone-rod dystrophy, in which intravitreal injections of CNTF had beneficial effects (Chong, N.H., Alexander, R.A., Waters, L., Barnett, K.C., Bird, A.C., and Luthert, P.J. (1999) Repeated injections of a ciliary neurotrophic factor analogue leading to long-term photoreceptor survival in hereditary retinal degeneration. Invest Ophthalmol Vis Sci 40: 1298-1305).

On the other hand, Schlichtenbrede, F.C. et al. 2003 (Gene Therapy, 10:523-7) disclose that the intraocular treatment with CNTF (ciliary neurotrophic factor) may result in unwanted side effects. In order to avoid this, they suggest the temporary administration of CNTF by the used of an inducible promoter or a pharmacological slow release device with later removal of the ocular insert.

Another approach has been the use of gene therapy vectors to express survival factors in retinas of mice or rats with retinal degeneration. In two mouse models, rd and Prph2Rd (recessive mutation in peripherin) (Travis, G.H., Brennan, M.B., Danielson, P.E., Kozak, C.A., and Sutcliffe, J.G. (1989) Identification of a photoreceptor-specific mRNA encoded by the gene responsible for retinal degeneration slow (rds). Nature 338: 70-73; Connell, G., Bascom, R., Molday, L., Reid, D., McInnes, R.R., and Molday, R.S. (1991) Photoreceptor peripherin is the normal product of the gene responsible for retinal degeneration in the rds mouse. Proc Natl Acad Sci US A 88: 723-726.), subretinal injections of adenoviral vectors encoding a secretable form of CNTF delayed photoreceptor death (Cayouette, M., and Gravel, C. (1997) Adenovirus-mediated gene transfer of ciliary neurotrophic factor can prevent photoreceptor degeneration in the retinal degeneration (rd) mouse. Hum Gene Ther 8: 423-430; Cayouette, M., Behn, D., Sendtner, M., Lachapelle, P., and Gravel, C. (1998) Intraocular gene transfer of ciliary neurotrophic factor prevents death and increases responsiveness of rod photoreceptors in the retinal degeneration slow mouse. J Neurosci 18: 9282-9293). For its part, transgenic BDNF expression in the retina, which was hardly active by intravitreal injections, delays neurodegeneration in Q344ter mice (Okoye, G., 25 Zimmer, J., Sung, J., Gehlbach, P., Deering, T., Nambu, H., Hackett, S., Melia, M., Esumi, N., Zack, D.J., and Campochiaro, P.A. (2003) Increased expression of brain-derived neurotrophic factor preserves retinal function and slows cell death from rhodopsin mutation or oxidative damage. J Neurosci 23: 4164-4172; Sung, C.H., Makino, C., Baylor, D., and Nathans, J. (1994) A rhodopsin gene mutation responsible for autosomal dominant retinitis pigmentosa results in a protein that is defective in localization to the photoreceptor outer segment. J Neurosci 14: 5818-5833). These results also show that, apart from technical problems, an isolated intravitreal injection can be insufficient to have a neuroprotective effect. For its part, CNTF, which in addition does have a neuroprotective effect with a single injection, has proved to be counterproductive when its prolonged expression is induced by means of AAV vectors encoding the secretable form of CNTF, in Prph2Rd mice and two transgenic rats with retinal degeneration (Liang, F.Q., Aleman, T.S., Dejneka, N.S., Dudus, L., Fisher, K.J., Maguire, A.M., Jacobson, S.G., and Bennett, J. (2001) Long-term protection of retinal structure but not function using RAAV.CNTF in animal models of retinosis pigmentaria. Mol Ther 4: 461-472) because the photoreceptor function worsens according to an analysis by ERG.

Taking into account all the strategies set forth above, the present invention provides a practical solution compared to systems used up until now. Survival functions of insulin have been shown before which are different from its metabolic function in chicken embryos, because insulin in its proinsulin precursor form is expressed during the development before the existence of a pancreatic outline. During the development of the nervous system, proinsulin regulates multiple cell processes. It is a survival factor in early embryos, as was verified in a study inhibiting the expression of the proinsulin gene or its receptor by means of using antisense oligonucleotides (Morales, A.V., Serna, J., Alarcon, C., de la Rosa, E.J., and de Pablo, F. (1997) Role of prepancreatic (pro)insulin and the insulin receptor in prevention of embryonic apoptosis. Endocrinology 138: 3967-3975). Its blocking by means of antibodies also increases the number of apoptotic cells in chicken embryo retina (Diaz, B., Serna,J., De Pablo,F., and de la Rosa, E. J. (2000) In vivo regulation of cell death by embryonic(pro) insulin and the insulin receptor during early retinal neurogenesis. Development 127: 1641-1649), whereas its exogenous addition to the embryo reduces the number of apoptotic cells (Hernandez-Sanchez, C., Mansilla, A., de la Rosa, E.J., Pollerberg, G.E., Martinez-Salas, E., and de Pablo, F. (2003) Upstream AUGs in embryonic proinsulin mRNA control its low translation level. Embo J 22: 5582-5592). The molecule that can be isolated from chicken embryos is proinsulin, the primary product of the gene translation, the metabolic activity of which is small, about 5-10% of the activity of insulin.

Corrochano, S. et al. 2005 ('Insulina y muerte celular programada: expresi6n y efectos durante la neurogenesis en la retina de rat6n'. XI CONGRESO DE LA SOCIEDAD ESPAÑOLA DE BIOLOGÍA CELULAR) disclose the ability of proinsulin and insulin for preventing apoptosis in a growth factor-deprived organotypic culture of E5 chicken retina.

In chronic neurodegenerative diseases, neurons and/or glial cells die progressively. The symptoms of the disease normally appear when quite a few cells have died. It is thus important to determine the effective molecules favoring cell survival for maintaining a relatively normal visual function. Although there are many types of neurodegenerative diseases, each with a different etiology, all of them present a final death of the affected cells. This death is a programmed death type, there being different types of apoptotic or non-apoptotic death. Unlike acute damage, in which the cells are initially in good condition before the damage, in chronic diseases the cells have an intrinsic damage which will make them die at a certain time when they can no longer support the damage and/or cannot carry out the function which they must perform.

### Description of the Invention

### Brief Description

An object of the present invention is formed by the use of a compound that induces the activity of proinsulin, hereinafter use of an inducer compound of the present invention, wherein said compound is selected from the group consisting of
a. a nucleotide sequence allowing the expression of a neuroprotective protein or peptide and which is formed by one or several nucleotide sequences belonging to the group of
   i. a nucleotide sequence formed by the nucleotide sequence encoding human proinsulin SEQ ID NO 1,
   ii. a nucleotide sequence which has a degree of identity of at least 95% to the sequence of i) and encoding a protein mimicking the activity of human proinsulin and,
   iii. a nucleotide sequence comprising any one sequence belonging to i) and/or ii);
b. an isolated human eukaryotic cell which is genetically modified and transformed by means of the human proinsulin nucleotide sequence SEQ ID NO:1 and comprises the construct or expression vector and can suitably release the proinsulin protein to the extracellular medium and
c. a protein or peptide having neuroprotective activity and comprising one or several amino acid sequences belonging to the group of
   i. an amino acid sequence formed by the human proinsulin amino acid sequence SEQ ID NO: 2,
   ii. an amino acid sequence which has a degree of identity of at least 95% to the sequence of i) and mimics the the neuroprotective activity of human proinsulin and,
   iii. an amino acid sequence comprising any one sequence belonging to: i) and/or ii) for the preparation of a medicinal product or pharmaceutical composition for the prevention and treatment of retinitis pigmentosa. In a preferred aspect of the invention, said pharmaceutical composition is suitable for its systemic or local sustained administration.

A particular object of the invention is formed by the use of a compound that induces the activity of proinsulin in which the inducer compound is a nucleotide sequence, hereinafter proinsulin nucleotide sequence of the present invention, which allows the expression of a neuroprotective protein or peptide, and which is formed by one or several nucleotide sequences belonging to the following group:
i) a nucleotide sequence formed by the nucleotide sequence encoding human proinsulin SEQ ID NO 1,
ii) a nucleotide sequence which has a degree of identity of at least 95% to the sequence of i) and encoding a protein mimicking the activity of human proinsulin and
iii) a nucleotide sequence comprising any one sequence belonging to: i), ii).

A particular embodiment of the present invention is formed by the use of a compound that induces the activity of proinsulin wherein the nucleotide sequence is formed by the SEQ ID NO 1 encoding human proinsulin.

Another particular object of the present invention is formed by the use of a compound that induces the activity of proinsulin wherein the nucleotide sequence of iii) is an expression vector, hereinafter use of the proinsulin expression vector of the invention, comprising a nucleotide sequence or a genetic construct encoding a proinsulin protein which can induce neuroprotection.

Another particular object of the present invention is formed by the use of a compound that induces the activity of proinsulin in which the inducer compound is preferably an isolated human eukaryotic cell, hereinafter use of proinsulin cells of the invention, which is genetically modified and transformed by means of the human proinsulin nucleotides sequence SEQ ID NO: 1 and comprises the construct or expression vector and can suitably release the proinsulin protein to the extracellular medium.

Another particular object of the invention is formed by the use of a compound that induces the activity of proinsulin in which the inducer compound is a protein or peptide, hereinafter use of the proinsulin protein of the present invention, having neuroprotective activity and comprising one or several amino acid sequences belonging to the following group:
i) an amino acid sequence formed by the human proinsulin amino acid sequence SEQ ID NO 2,
ii) an amino acid sequence which has a degree of identity of at least 95% to the sequence of i), and mimics the neuroprotective activity of human proinsulin, and
iii) an amino acid sequence comprising any one sequence belonging to: i) and/or ii).

Another particular embodiment of the present invention is formed by the use of an inducer compound of the invention in which the inducer compound is the human proinsulin protein SEQ ID NO 2.

Another object of the present invention is formed by a pharmaceutical composition or medicinal product for use in the treatment of retinitis pigmentosa, characterized in that it comprises a compound that induces the activity of proinsulin in a therapeutically effective amount together with one or more pharmaceutically acceptable adjuvants and/or carriers wherein the compound is selected from the group of
a. a nucleotide sequence allowing the expression of a neuroprotective protein or peptide and which is formed by one or several nucleotide sequences belonging to the group of
   i. a nucleotide sequence formed by the nucleotide sequence encoding human proinsulin SEQ ID NO 1,
   ii. a nucleotide sequence which has a degree of identity of at least 95% to the sequence of i) and encoding a protein mimicking the activity of human proinsulin and,
   iii. a nucleotide sequence comprising any one sequence belonging to i) and/or ii);
b. an isolated human eukaryotic cell which is genetically modified and transformed by means of the human proinsulin nucleotide sequence SEQ ID NO 1 and comprises the construct or expression vector and can suitably release the proinsulin protein to the extracellular medium;
c. a protein or peptide having neuroprotective activity and comprising one or several amino acid sequences belonging to the group of
   i. an amino acid sequence formed by the human proinsulin amino acid sequence SEQ ID NO 2,
   ii. an amino acid sequence which has a degree of identity of at least 95% to the sequence of i), and mimics the neuroprotective activity of human proinsulin and,
   iii. an amino acid sequence comprising any one sequence belonging to: i) and/or ii):

A particular embodiment of the invention is formed by a pharmaceutical composition of the invention in which the compound that induces the activity of the proinsulin is one or several nucleotide sequences belonging to the following group:
i) a nucleotide sequence formed by the nucleotide sequence encoding human proinsulin SEQ ID NO 1,
ii) a nucleotide sequence which has a degree of identity of at least 95% to the sequence of i) and encoding a protein mimicking the activity of human proinsulin and
iii) nucleotide sequence comprising any one sequence belonging to: i) and/or i).

Another particular embodiment of the present invention is formed by the pharmaceutical composition of the invention in which the nucleotide sequence is formed by SEQ ID NO 1, encoding human proinsulin.

Another particular embodiment of the present invention is formed by.a pharmaceutical composition of the invention in which the nucleotide sequence is a human proinsulin expression vector.

Another particular object of the present invention is formed by a pharmaceutical composition of the invention in which the compound that induces the activity of proinsulin is a protein or a peptide encoded by the proinsulin sequence, genetic construct or vector of the invention.

A particular embodiment of the invention is formed by a pharmaceutical composition of the invention in which the protein or peptide that induces the activity of proinsulin belongs to the following group:
a) an amino acid sequence formed by the human proinsulin amino acid sequence SEQ ID NO 2,
b) an amino acid sequence which has a degree of identity of at least 95% to the sequence of i) and mimics the neuroprotective activity of human proinsulin andJiii) an amino acid sequence comprising any one sequence belonging to: a: i) and/or or ii).

Another particular embodiment of the present invention is formed by the pharmaceutical composition of the invention in which the amino acid sequence is formed by human proinsulin (SEQ ID NO 2).

Another particular object of the present invention is formed by a pharmaceutical composition of the invention in which the compound that induces the activity of proinsulin is preferably an isolated human cell, and more preferably a central nervous system cell transformed by the proinsulin sequence, genetic construct or expression vector of the invention.

It is also disclosed herein the pharmaceutical composition of the invention, hereinafter use of the pharmaceutical composition of the invention, in a method of treatment or prophylaxis of a mammal, preferably a human being, affected by a neurodegenerative disease, disorder or pathology of the central or peripheral nervous systems affecting human beings, in which programmed cell death occurs, consisting of administering said therapeutic composition in a suitable dose which allows reducing said neurodegeneration.

### Detailed Description

Taking into account that most neurodegenerative conditions, particularly retinitis pigmentosa, do not have an efficient and/or effective treatment, the present invention provides an alternative solution.

The present invention is based on the fact that proinsulin - a growth factor of the insulin family normally known as being the precursor form of insulin - is furthermore a cell survival factor in chronic neurodegeneration process, particularly in retinal neurodegeneration processes taking place in retinitis pigmentosa.

rd10 (*Pdeb*^{*rd10*/*rd10*}) and rd1 (*Pdeb*^{*rd1*/*rd1*}) mice, carrying a recessive homozygous mutation in the rod-specific cyclic GMP phosphodiesterase enzyme gene, which causes an alteration in the function of this enzyme, which leads to progressive photoreceptor cell death, and the subsequent secondary degeneration of the remaining retinal cell types, mainly the cones, have been chosen as the retinal degeneration model.

Two lines of transgenic mice expressing human proinsulin protein and recessive homozygotes for the rd10 mutation, Proins/rd10^{-/-} mice have been generated (Example 1.1). These Proins/rd10^{-l-} mice with retinal degeneration constitutively produce human proinsulin in striated muscle - which is not processed to insulin - which is detected in serum and is not subject to the normal regulation of the pancreas due to glucose levels. This causes the animal to have sustained circulating human proinsulin levels, without depending on the dose, on the condition of the product before administration - because as it is from an endogenous production it is not degraded such as a commercial product-, and on the form and time of the injection. This has allowed not conducting pharmacokinetics studies to see the way of administering it.

The presence of human proinsulin in muscle and serum in Proins/rd10^{-/-} mice of both lines was verified with an ELISA detection kit against human proinsulin. Furthermore, blood sugar was measured and it was verified that proinsulin did not have unwanted metabolic effects. In addition, it has been verified by means of subcutaneous injection of human proinsulin that said proinsulin can reach the neural retina (proinsulin identification by means of the ELISA kit in retina extracts), which means that it can pass through the blood-retina barrier.

The effects of hyperproinsulinemia of Proins/rd10^{-/-} mice in retinal neurodegeneration were identified in several ways. The condition of the retina was first observed histologically (Example 1). In transgenic mice for human proinsulin and homozygotes for rd10, the degeneration is delayed and it has been verified that at P32, a higher number of rods, cones and synaptic connections are maintained in the retina and the condition of the retina is better (Example 1).

The fact that the condition of retina is maintained better for more time is beneficial. If apart from slowing down the cell death process in actually damaged cells (the rods of the retina), the rest of the retina is maintained in a better condition, such as for example by preventing the death of the cones which do not have intrinsic damage but rather which degenerate in a secondary manner, several aspects of the disease are being improved. In this specific case, if the cones are maintained for more time, although the rods end up being degenerated, daytime vision is maintained, albeit night vision is lost, and that means quality of life in a patient with retinitis pigmentosa.

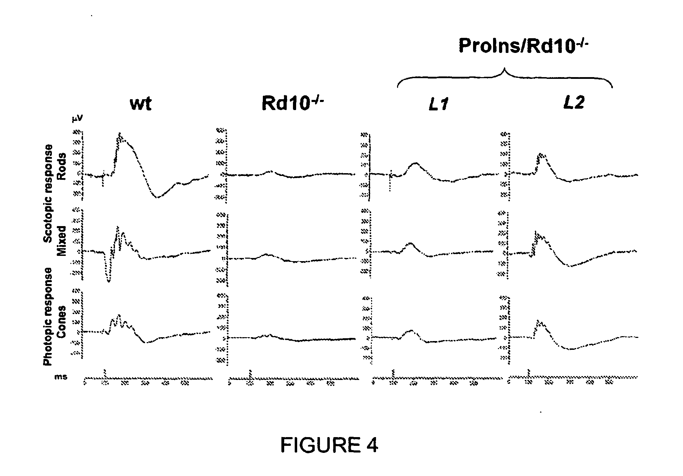

The visual function was subsequently analyzed with the five standardized types of electroretinograms throughout the degenerative process, comparing the transgenic mice with rd10 controls, and it was verified that in Proins/rd10^{-/-} mice, they still have visual function at P55, which visual function disappears at P35 in rd10 mice, whereby it can be concluded that the visual response is better and is more extended over time than in Proins/rd10^{-/-} mice (Example 2). Furthermore, transgenic Proins/rd10^{-/-} mice have ERGs comparable to healthy mice, al least in photopic (daytime) vision, at P30 (Example 2, Figure 4).

In addition, in the present invention it is observed that the use of proinsulin in a form of administration allowing physiological levels that are sustained over time allows proinsulin to exert its neuroprotective function (see Example 1.4), in contrast to that observed with isolated subcutaneous and intravitreal administrations of proinsulin that have been tested but were not successful in improving retinal neurodegeneration, probably because levels that were stable and sustained over time were not obtained (Examples 3 and 4).

Another additional advantage is that proinsulin does not have the metabolic effects of insulin and therefore, it can be administered to patients whose glucose metabolism is not altered. Although insulin also has antiapoptotic activity in *in vitro* studies, its normal metabolic activity makes its use for a treatment such as the one described herein unviable. The fact that circulating serum proinsulin achieves this rescue indicates that it is a good treatment because it makes it easy to administer. The application of proinsulin would be both a preventive and suppressive treatment of neurodegenerative diseases, because it prevents the death of damaged neurons.

In summary, chronic serum proinsulinemia levels (1-15 pM), obtained by means of transgenic human proinsulin expression controlled by the myosin light chain promoter, which could also be obtained by means of other forms of sustained administration, subcutaneous injection of human proinsulin carried on slow release supports or expression vectors approved for gene therapy, for example, can reach the retina and reduce photoreceptor neurodegeneration in the genetic rd10 mouse model.

Therefore, an object of the present invention is formed by the use of a compound that induces the activity of proinsulin, hereinafter use of an inducer compound of the present invention, wherein said compound is selected from the group consisting of
a. a nucleotide sequence allowing the expression of a neuroprotective protein or peptide and which is formed by one or several nucleotide sequences belonging to the group of
   i. a nucleotide sequence formed by the nucleotide sequence encoding human proinsulin SEQ ID NO 1,
   ii. a nucleotide sequence which has a degree of identity of at least 95% to the sequence of i) and encoding a protein mimicking the activity of human proinsulin and,
   iii. a nucleotide sequence comprising any one sequence belonging to i) and/or ii);
b. an isolated human eukaryotic cell which is genetically modified and transformed by means of the human proinsulin nucleotide sequence SEQ ID NO:1 and comprises the construct or expression vector and can suitably release the proinsulin protein to the extracellular medium and
c. a protein or peptide having neuroprotective activity and comprising one or several amino acid sequences belonging to the group of
   i. an amino acid sequence formed by the human proinsulin amino acid sequence SEQ ID NO: 2,
   ii. an amino acid sequence which has a degree of identity of at least 95% to the sequence of i) and mimics the neuroprotective activity of human proinsulin and,
   iii. an amino acid sequence comprising any one sequence belonging to: i) and/or ii) for the preparation of a medicinal product or pharmaceutical composition for the prevention and treatment of retinitis pigmentosa. In a preferred embodiment of the present invention, the pharmaceutical composition further comprises a suitable carrier for systemically or locally administering the pharmaceutical composition in a sustained manner.

In the present invention, it is understood that a pharmaceutical composition is suitable for its systemic or local sustained administration when said composition is pharmaceutically carried, compressed or formulated such that it is constantly released into the body, being maintained at an effective dose in the target tissue for an extended time period. In one aspect of the invention, any carrier suitable for administering the pharmaceutical composition in a sustained manner so as obtein chronic proinsulinemia level of 1-15p M, for example although not limited to polymers or patches, would be comprised within the scope of protection of the present invention.

As used in the present invention, the term "compound that induces the activity of proinsulin" relates to a molecule mimicking, increasing the intensity or extending the duration of the neuroprotective activity of human proinsulin protein. An activator compound can be formed by a chemical molecule, a peptide, a protein or a nucleotide sequence, as well as those molecules allowing the expression of a nucleotide sequence encoding a protein with neuroprotective activity.

As used in the present invention, the term "neuroprotective activity" relates to the reduction of the programmed cell death process of cells that are primarily affected in the neurodegenerative disease, and/or of the cells that are secondarily affected by neurodegeneration, and/or to the enhancement of the neurofunctional activity of the remaining cells.

As used in the present invention, the term "neurodegenerative disease" relates to a disease, disorder or pathology belonging, among others by way of illustration and without limiting the scope of the invention, to the following group: Alzheimer's disease, Parkinson's disease, multiple sclerosis, retinitis pigmentosa, dementia with Lewy bodies, amyotrophic lateral sclerosis, spinocerebellar atrophies, frontotemporal dementia, Pick's disease, vascular dementia, Huntington's disease, Baten's disease, spinal cord injury, macular degeneration and glaucoma.

Thus, a particular object of the invention is formed by the use of a compound that induces the activity of proinsulin in which the inducer compound is a nucleotide sequence, hereinafter use of the proinsulin nucleotide sequence of the present invention, allowing the expression of a neuroprotective protein or peptide, and which is formed by one or several nucleotide sequences belonging to the following group:
i) a nucleotide sequence formed by the nucleotide sequence encoding human proinsulin SEQ ID NO 1,
ii) a nucleotide sequence which has a degree of identity of at least 95% to the sequence of i) and encoding a protein mimicking the activity of human proinsulin, and
iii) a nucleotide sequence comprising any one sequence belonging to: i) and/or ii).

In the sense used in this description, the term similar" intends to include any nucleotide sequence which can be isolated or constructed based on the sequence shown in this specification, for example, by means of introducing conservative and non-conservative nucleotide substitutions, including the insertion of one or more nucleotides, the addition of one or more nucleotides in any of the ends of the molecule or the deletion of one or more molecules in any end or inside the sequence, and which allows encoding a peptide or protein which can mimic the activity of human proinsulin (SEQ ID NO 2).

Based on the information described in the present invention and in the state of the art, a person skilled in the art can isolate or construct a nucleotide sequence similar to those described in the present invention for its subsequent use.

A similar nucleotide sequence is generally substantially homologous to the aforementioned nucleotide sequence. In the sense used herein, the expression "substantially homologous" means that the nucleotide sequences in question have a degree of identity of at least 95%. The preferred form of the nucleotide sequence to be used is the human proinsulin nucleotide sequence (SEQ ID NO 1) and derivatives thereof.

As used in the present invention, the term "nucleotide sequence" relates to a DNA, cDNA or mRNA sequence.

A particular embodiment of the present invention is formed by the use of a compound that induces the activity of proinsulin wherein the nucleotide sequence is formed by the SEQ ID NO 1 encoding human proinsulin.

The nucleotide sequence defined in section iii) corresponds to a gene construct and to a gene expression vector allowing the expression of a proinsulin protein. In the case of the gene construct, proinsulin gene construct of the invention, it can also comprise, if necessary and to allow a better isolation, detection or secretion to the exterior of the cell of the expressed peptide, a nucleotide sequence encoding a peptide which can be used for purposes of isolation, detection or secretion of said peptide. Therefore, another particular object of the present invention is formed by a gene construct comprising, in addition to the proinsulin nucleotide sequence of the invention, any other nucleotide sequence encoding a peptide or peptide sequence allowing the isolation, detection or the secretion to the exterior of the cell of the expressed peptide, for example, by way of illustration and without limiting the scope of the invention, a polyhistidine (6xHis) sequence, a peptide sequence which can be recognized by a monoclonal antibody (for its identification, for example), or any other sequence which is useful for purifying the fusion protein resulting from immunoaffinity chromatography: tag peptides such as c-myc, HA, E-tag) (Using antibodies: a laboratory manual. Ed. Harlow and David Lane 10 (1999). Cold Spring Harbor Laboratory Press. New York. Chapter: Tagging proteins. Pp. 347-377).

The previously described nucleotide sequence and the gene construct can be isolated and obtained by a skilled person by means of using teghniques that are widely known in the state of the art (Sambrook et al. "Molecular cloning, a Laboratory Manual 2nd ed., Cold Spring Harbor Laboratory Press, N.Y., 1989 vol 1-3). Said nucleotide sequences can be integrated in a gene expression vector which allows regulating the expression thereof in suitable conditions inside the cells.

Therefore, another particular object of the present invention is formed by the use of a compound that induces the activity of proinsulin wherein the nucleotide sequence of iii) is an expression vector, hereinafter use of the proinsulin expression vector of the invention, comprising a nucleotide sequence or a gene construct encoding a proinsulin protein which can induce neuroprotection. An example of a particular embodiment is formed by the use of an expression vector prepared in the present invention in which the expression is regulated by means of a muscle-specific promoter and the nucleotide sequence SEQ ID NO 1 (see Example 1).

In addition to the nucleotide sequence or the genetic construct described in the invention, an expression vector generally comprises a promoter directing its transcription (for example, pT7, plac, ptrc, ptac, pBAD, 5 ret, etc.), preferably a tissue promoter, to which it is operatively linked, and other necessary or suitable sequences controlling and regulating said transcription and where appropriate, the translation of the product of interest, for example, transcription initiation and termination signals (tlt2, etc.), polyadenylation signal, replication origin, ribosome binding sequences (RBS), sequences encoding transcriptional regulators (enhancers), transcriptional silencers, repressors, etc. Examples of suitable expression vectors can be selected according to the conditions and needs of each specific case from expression plasmids, viral vectors (DNA or RNA), cosmids, artificial chromosomes, etc. which can further contain markers that can be used to select the cells transfected or transformed with the gene or genes of interest. The choice of the vector will depend on the host cell and of the type of use to be carried out. Therefore, according to a particular embodiment of the present invention said vector is a plasmid or a viral vector. Said vector can be obtained by conventional methods known by the persons skilled in the art in the same way as different widely known methods - chemical transformation, electroporation, microinjection, etc. - described in different manuals [Sambrook, J., Fritsch, E.F., and Maniatis, T. (1989). Molecular cloning: a laboratory manual, 2nd ed. Cold Spring Harbor Laboratory, Cold Spring Harbor, N.Y.] can be used for the transformation of eukaryotic cells and microorganisms. One strategy could be to use lentiviruses to infect target cells, as is already being attempted in other types of therapies (Ralph GS, Binley K, Wong LF, Azzouz M, Mazarakis ND (2006) Gene therapy for neurodegenerative and ocular diseases using lentiviral vectors. Clin Sci (Lond) 110: 37-46).

Gene expression systems can or cannot allow the integration of new genetic material in the genome of the host cell. The nucleotide sequence, the gene construct or the proinsulin expression vector can thus be used as a medicinal product for protecting human cells, preferably human neurons and/or glial cells affected by a neurodegenerative alteration, in a process of gene therapy prophylaxis and treatment of a human being affected by a disease presenting neuronal and/or glial alterations. Once these gene expression systems have been administered to a human being affected by a neurodegenerative disease, they can be generally or specifically introduced in tissue cells where, once they have been integrated in the cell genome, they allow the expression of a proinsulin protein, which once it has been secreted to the extracellular medium, reaches the central nervous system where it could carry out its neuroprotective action (see examples).

In addition, these gene expression systems can also be used to transform human cells outside the human body, autologous or heterologous in relation to the potential recipient, these cells becoming compounds that induce proinsulin once they are administered to a human being suffering from a neurodegenerative disease because they express and release proinsulin protein with neuroprotective activity for human neurons and/or glial cells.

Thus, another particular object of the present invention is formed by the use of a compound that induces the activity of the proinsulin in which the inducer compound is a preferably isolated human eukaryotic cell, hereinafter proinsulin cells of the invention, which is genetically modified and comprises the proinsulin nucleotide sequence SEQ ID NO 1, construct or expression vector of the invention and can suitably release the proinsulin protein to the extracellular medium.

These cells can be transformed, infected or transfected by means of said nucleotide sequences by genetic engineering techniques known by a person skilled in the art. [Sambrook, J., Fritsch, E.F., and Maniatis, T. (1989). Molecular cloning: a laboratory manual, 2nd ed. Cold Spring Harbor Laboratory]. The biopharmaceutical tools and gene therapy processes are sufficiently known by a person skilled in the art such that they can be developed without excessive effort with the information described in the present invention.

Another particular embodiment would be the use of an isolated human cell transformed by means of the human proinsulin nucleotide sequence (SEQ ID NO 1), from different cell strains, preferably from the central nervous system more preferably a neuron which can be used as cells regenerating human tissue.

Furthermore, another particular object of the invention is formed by the use of a compound that induces the activity of proinsulin, in which the inducer compound is a protein or peptide, hereinafter use of the proinsulin protein of the present invention, having neuroprotective activity, and comprising one or several amino acid sequences belonging to the following group:
i) an amino acid sequence formed by the human proinsulin amino acid sequence SEQ ID NO 2,
ii) a similar amino acid sequence which has at degree of identity of at least 95% to the sequence of i), and mimics the neuroprotective activity of human proinsulin, and
iii) an amino acid sequence comprising any one sequence belonging to: i) and/or ii).

In the sense used herein, the term "similar" intends to include any amino acid sequence which can be isolated or constructed based on the sequence shown in the present specification, for example by means of introducing conservative or non-conservative amino acid substitutions, including the insertion of one or more amino acids, the addition of one or more amino acids in any of the ends of the molecule or the deletion of one or more amino acids in any end or inside the sequence, and mimicking the neuroprotective activity of human proinsulin.

Based on the information described in the present invention, a person skilled in the art can isolate or construct an amino acid sequence similar to those described in the present invention.

A similar amino acid sequence is generally substantially homologous to the aforementioned amino acid sequence. In the sense used herein, the expression "substantially homologous" means that the amino acid sequences in question have a degree of identity of at least 95%.

Another particular embodiment of the present invention is formed by the use of an inducer compound of the invention in which the inducer compound is human proinsulin protein (SEQ ID NO 2).

Another object of the present invention is formed by a phatmaceutical composition or medicinal product for use in the treatment of retenitis pigmentosa , hereinafter pharmaceutical composition of the preseat invention, characterized in that it comprises a compound that induces the activity of proinsulin of the invention, in a therapeutically effective amount together with one or more pharmaceutically acceptable adjuvants and/or carriers wherein said compound is selected from the group consisting of
a. a nucleotide sequence allowing the expression of a neuroprotective protein or peptide and which is formed by one or several nucleotide sequences belonging to the group of
   i. a nucleotide sequence formed by the nucleotide sequence encoding human proinsulin SEQ ID NO 1,
   ii. a nucleotide sequence which has a degree of identity of at least 95% to the sequence of i)and encoding a protein mimicking the activity of human proinsulin and,
   iii. a nucleotide sequence comprising any one sequence belonging to i) and/or ii);
b. an isolated human eukaryotic cell which is genetically modified and transformed by means of the human proinsulin nucleotide sequence SEQ ID NO:1 and comprises the construct or expression vector and can suitably release the proinsulin protein to the extracellular medium
c. a protein or peptide having neuroprotective activity and comprising one or several amino acid sequences belonging to the group of
   i. an amino acid sequence formed by the human proinsulin amino acid sequence SEQ ID NO: 2,
   ii. an amino acid sequence which has a degree of identity of at least 95% to the sequence of i) and mimics the neuroprotective activity of human proinsulin and,
   iii. an amino acid sequence comprising any one sequence belonging to: i) and/or ii.

The pharmaceutically acceptable adjuvants and carriers which can be used in said compositions are the adjuvants and carriers known by persons skilled in the art and commonly used in preparing therapeutic compositions.

In a particular embodiment, the pharmaceutical composition of the invention, is suitable for maintaining a systemic or local sustained administration of the inducer compound so as to obtain chronic proinsulinemia levels of 1-15 pM.

In the sense used herein, the expression "therapeutically effective amount" relates to the amount of agent or compound which can develop neuroprotection, calculated to produce the desired effect and which will generally be determined, among other reasons, by the own characteristics of the compounds, including the age, condition of the patient, severity of the alteration or disorder, and the route and frequency of administration.

In another particular embodiment, said therapeutic composition is prepared in the form of a solid form or aqueous suspension, in a pharmaceutically acceptable diluent. The therapeutic composition provided by this invention can be administered by any suitable method of administration, for which said composition will be formulated in the suitable dosage form for the chosen method of administration. In a particular embodiment, the therapeutic composition provided by, this invention is administered parenterally, orally, by nasal inhalation, intraperitoneally, subcutaneously, etc. A review of the different dosage forms for administering medicinal products and of the excipients necessary for obtaining them can be found, for example, in the "Tratado de Farmacia Galénica", C. Faulí i Trillo, 1993, Luzán 5, S.A. Ediciones, Madrid.

A particular embodiment of the invention is formed by a pharmaceutical composition of the invention in which the compound that induces the activity of proinsulin is one or several nucleotide sequences belonging to the following group:
i) a nucleotide sequence formed by the nucleotide sequence encoding human proinsulin SEQ ID NO 1,
i) a nucleotide sequence which has a degree of identity of at least 95% to the sequence of i)and encoding a protein mimicking the activity of human proinsulin, and
iii) a nucleotide sequence comprising any one sequence belonging to: i) and/or ii).

Another particular embodiment of the present invention is formed by the pharmaceutical composition of the invention in which the nucleotide sequence is formed by SEQ ID NO 1, encoding human proinsulin.

Another particular embodiment of the present invention is formed by a pharmaceutical composition of the invention in which the nucleotide sequence is a human proinsulin expression vector.

Another particular object of the present invention is formed by a pharmaceutical composition of the invention in which the compound that indices the activity of proinsulin is a protein or peptide encoded by the proinsulin sequence, genetic construct or vector of the invention.

A particular embodiment of the invention is formed by a pharmaceutical composition of the invention in which the protein or peptide that induces the activity of proinsulin belongs to the following group:
i) an amino acid sequence formed by the human proinsulin amino acid sequence SEQ ID NO 2,
ii) an amino acid sequence which has a degree of identity of at least 95% to the sequence of i) and mimics the neuroprotective activity of human proinsulin, and
iii) an amino acid sequence comprising any one sequence belonging to: i) and/or ii).

Another particular embodiment of the present invention is formed by the pharmaceutical composition of the invention in which the amino acid sequence is formed by human proinsulin (SEQ ID NO 2).

Another particular object of the present invention is formed by a pharmaceutical composition of the invention in which the compound that induces the activity of proinsulin is preferably an isolated human cell, preferably a central nervous systems cell, transformed by the proinsulin sequence, construct or expression vector of the invention.

Another particular object of the present invention is formed by the pharmaceutical composition of the invention for use in the treatment of retinitis pigmentosa, characterized in that the isolated human eukayotic cell which is genetically modified and transformed by means of the human proinsulin nucleotide sequence of SEQ ID NO: 1 and comprises the construct or expression vector and can suitable release the proinsulin protein to the extracellular medium, is a central nervous system cell.

Another object of the invention is formed by the use of the pharmaceutical composition the invention, hereinafter use of the pharmaceutical composition of the invention, in a method of treatment or prophylaxis of a mammal, preferably a human being, affected by a neurodegenerative disease, disorder or pathology of the central or peripheral nervous systems affecting human beings, in which programmed cell death occurs, consisting of administering said therapeutic composition in a suitable dose which allows reducing said neurodegeneration.

The pharmaceutical composition of the present invention can be used in a method of treatment in an isolated manner or together with other pharmaceutical compounds.

Another particular object of the present invention is formed by the use of the pharmaceutical composition of the invention in a method of treatment of a neurodegenerative disease belonging to the following group: Alzheimer's disease, Parkinson's disease, multiple sclerosis, retinitis pigmentosa, dementia with Lewy bodies, amyotrophic lateral sclerosis, spinocerebellar atrophies, frontotemporal dementia, Pick's disease, vascular dementia, Huntington's disease, Baten's disease and spinal cord injury.

Another particular embodiment of the present invention is formed by the use of the pharmaceutical composition of the invention in a method of treatment of a neurodegenerative disease belonging to the following group: retinitis pigmentosa, macular degeneration and glaucoma.

### Brief Description of the Content of the Drawings

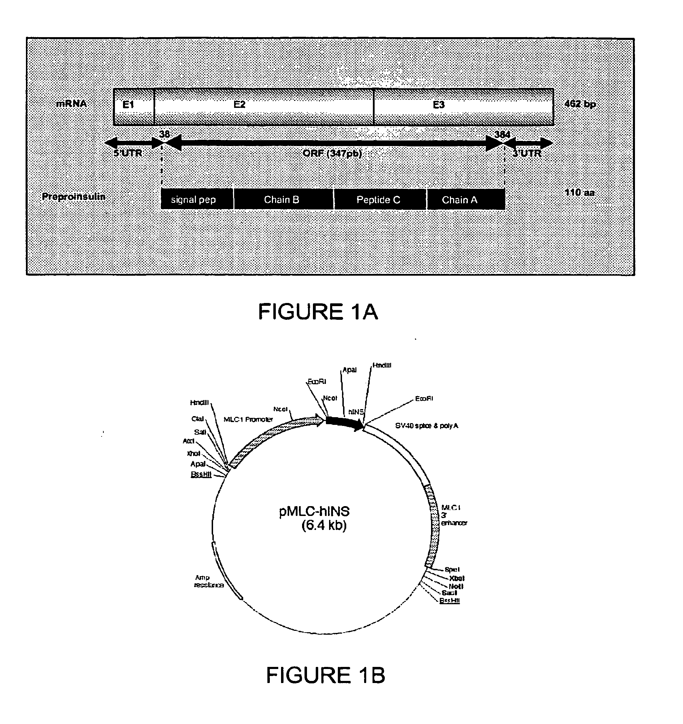

Figure 1 shows a schematic representation of the insert of cDNA of the human preproinsulin gene and of the plasmid pMLC-hIns. (A) A schematic representation of the DNA sequence which is inserted in the plasmid is shown. It corresponds to the cDNA of the human proinsulin gene. The diagram shows the mRNA of the 462 base pair (bp) gene which is formed by three exons (exon1= E1, exon2= E2, exon 3= E3). The inserted DNA sequence is the one which is translated, the 347 bp ORF (Open Reading Frame). The untranslated flanking areas (5'UTR and 3'UTR) are not inserted in the construct. The protein which is translated is the 110 amino acid (aa) preproinsulin. This protein consists of a signal peptide (signal pep.), chain B, peptide C and chain A. The signal peptide is eliminated, leaving the proinsulin molecule. (B) The plasmid contains the insert described in the previous section 1.A, encoding the 110 amino acid preproinsulin protein (thick area in black), under the transcriptional control of the constitutive muscular promoter MLC1 (Myosin Light Chain) of the striated muscle myosin light chain fibers. This plasmid is used to produce the two lines of transgenic human proinsulin producing mice which were crossed to reach homozygosis for rd10.

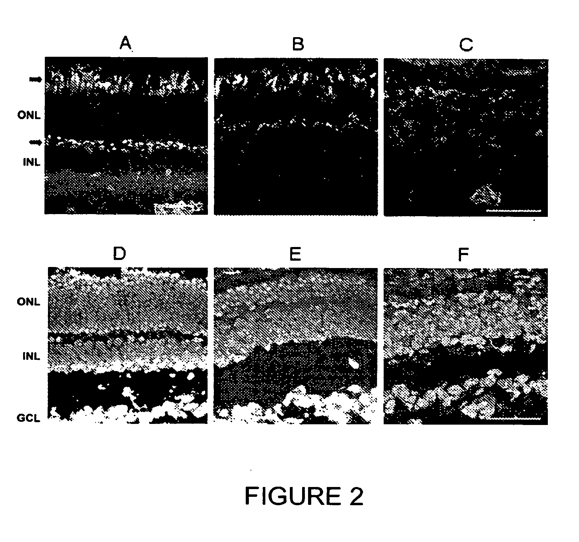

Figure 2 shows 12 µm cryostat eye sections of mice at P32, in which the condition of rods and cones showing the progress of degeneration with different markers can be observed. Wild-type control mouse (A and D), homozygous rd10 and transgenic mouse producing proinsulin, Proins/rd10^{-/-}, (B and E) and control homozygous rd10 mouse (C and F). The outer nuclear layer (ONL) in which the nuclei of the rods and cones are located can be seen by means of nuclear staining with DAPI. The inner nuclear layer (INL) is where the nuclei of bipolar, amacrine, horizontal and Müller cells are located. An agglutinin labeled with fluorochrome Alexa 488, the reaction of which shows the outer segments (upper arrow) and the synaptic feet of the cones (lower arrow), is used to label the cones. The bar represents 45 µm.

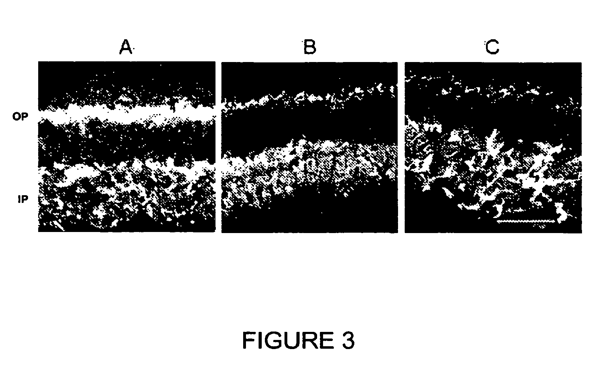

Figure 3 shows 12 µm eye cryostat sections of mice at P32, in which the condition of the synaptic connections can be observed. Wild-type control mouse (A), homozygous rd10 and transgenic mouse producing proinsulin, Proins/rd10^{-/-}, (B) and control homozygous rd10 mouse (C). The nuclear layers ONL, INL and the ganglion cell layer (GCL) are shown. The plexiform layers in which the synaptic connections occur, outer plexiform layer (OPL) and inner plexiform layer (IPL) are located between them. The figure shows the immunohistochemical labeling with the SV2 antibody generally labeling the synaptic connections. The bar represents 0.45 µm.
Figure 4 shows the results of the electroretinographic recordings carried out at P30. Wild-type mouse (wt), control Rd10^{-/-} mouse and Proins/Rd10^{-/-} mouse, both from line 1 (L1) and from line 2 (L2). Examples of the electroretinographic responses recorded in scotopic (night) condition, generated in rods (upper row) and mixed responses (intermediate row) are shown for each animal. The electroretinographic responses recorded in photopic (daytime) conditions, generated in cones (lower row) are also shown. The greater range of the responses obtained in Proins/rd10^{-/-} mice from both lines (L1 and L2) compared to the responses of Rd10^{-/-} mice should be noted.

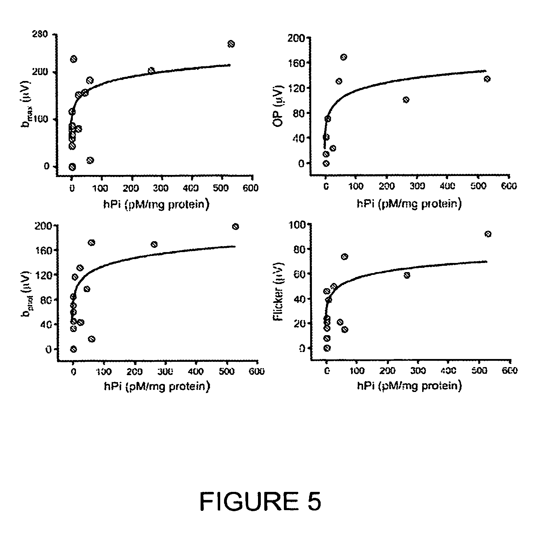

Figure 5 shows the correlation between the human proinsulin levels and the maintenance of vision parameters. The human proinsulin levels in Proins/rd10^{-/-} mice were determined at P32 in the quadriceps muscle of mice which had undergone a complete electroretinographic examination at P30. The respective values of proinsulin and of the different vision parameters follow hyperbolic curves. The amplitudes of the electroretinographic waves bₘₐₓ (recorded in response to 1.5 log cd.s.m⁻²), OP (oscillating potential), bₚₕₒₜ (recorded in response to 1.5 log cd.s.m⁻²), and the Flicker response are shown.

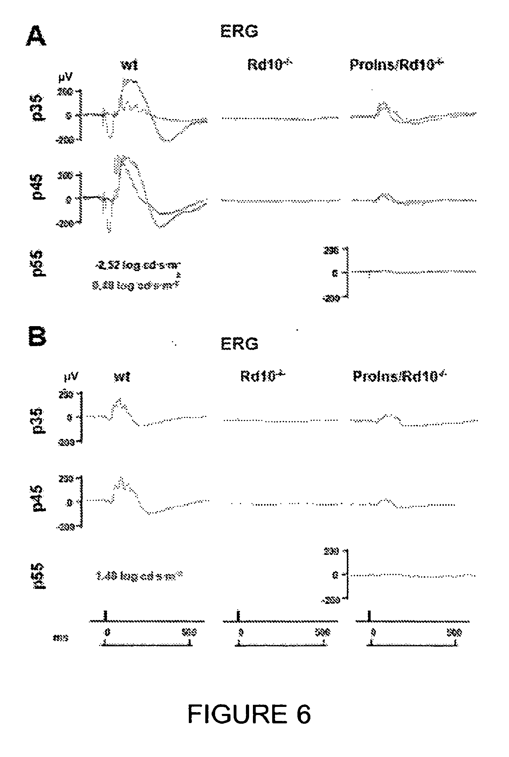

Figure 6 shows the results of the electroretinographic recordings carried out on different days. Wild-type mouse (wt), control Rd10^{-/-} mouse and Proins/Rd10^{-/-} mouse, on different days of postnatal development: P35, P45, P55. A shows examples of the responses recorded in scotopic conditions, exclusive for rods (-2.55 log cd.s.m⁻²), and mixed responses (1.48 log cd.s.m⁻²) for each type of animal and at the different times of postnatal development. B also shows examples of the responses recorded in photopic conditions, generated in cones (1.48 log cd.s.m⁻²) for each type of animal and at the different times of postnatal development.

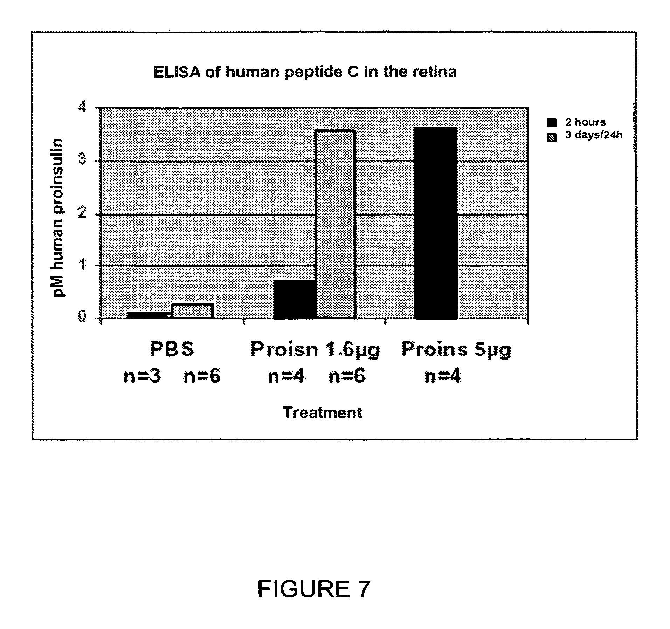

Figure 7 shows the presence of human proinsulin in the retina after its subcutaneous injection. Quantification of human proinsulin by ELISA in retinal extracts of C57B1/6 mice subcutaneously injected with the indicated amounts of proinsulin, 2 hours before preparing the extract, or daily, between P11 and P14, the last injection also being 2 hours before preparing the extract.

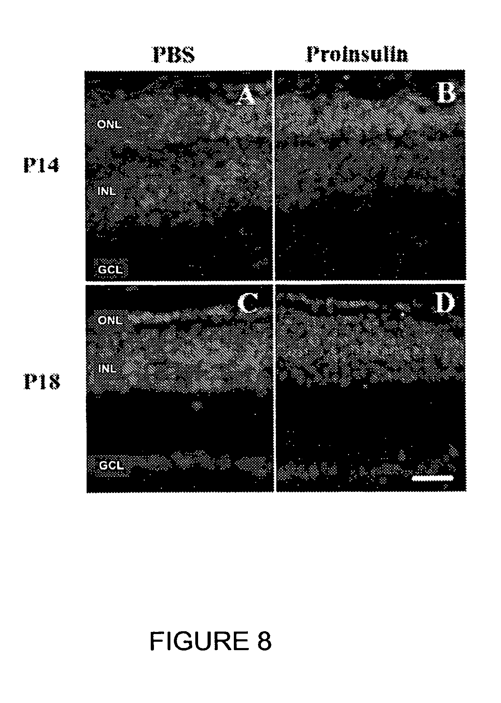

Figure 8 shows the effect of the subcutaneous injections of human proinsulin on the retinal histology of mice in neurodegeneration. Retinal sections of Rd1^{-/-} mice at P14 injected every 12 hours between P6 and P14 or P18 with human proinsulin (B and D) or carrier (A and C). The loss of photoreceptors in the outer nuclear layer can be observed by means of nuclear staining with DAPI in all the cases. ONL, outer nuclear layer; INL, inner nuclear layer, GCL, ganglion cell layer. The bar represents 25 µm.

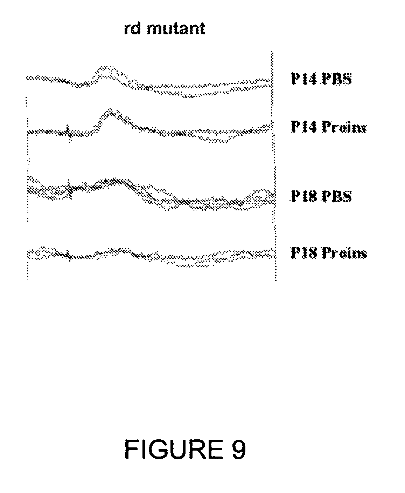

Figure 9 shows the effect of the subcutaneous injections of human proinsulin on the retinal histology of mice in neurodegeneration. Electroretinograms of Rd1^{-/-} mice injected every 12 hours between P6 and P14 or P18 with human proinsulin (Proins) or carrier (PBS). Electroretinograms of litter sibling rd mutant mice subjected to the different treatments are shown. The injection of proinsulin did not slow the visual function loss process.

### EMBODIMENTS

Following specific examples provided herein serve to illustrate the nature of the present invention. These examples are only included for illustrative purposes and must not be interpreted as limitations to the invention claimed herein.

### Example 1.- Human proinsulin can prevent retinal rod death and maintain the synaptic connections in transgenic Proins/rd10^{-/-} mice.

### 1.1.- Production of two transgenic lines producing human proinsulin and homozygotes for the rd10 mutation (Proins/rd10^{-/-})

After obtaining several lines of transgenic human proinsulin-producing mice under the control of the striated muscle myosin light chain (MLC1) constitutive promoter, the genetic baggage of which was 50% C57B1/6 and 50% SJL, they were successively crossed with homozygous rd10 mice (100% C57B1/6). The genetic baggage was thus homogenized until reaching a percentage greater than 95% C57B1/6 and Proins/rd10^{-/-} mice were obtained. This was achieved from the sixth backcross, obtaining two main lines, L1 and L2 (L2 animals have been used in the following examples, whereas the results described in Figure 4 ERG at P30 have also been carried out with L1 animals; the proinsulinemia and blood sugar analyses have also been carried out in both).

In the present invention, wild-type mice are understood as the mice used as control. It has no alteration. It is commercial and its name is C57B1/6J, from Jackson laboratories.

rd1 mice are understood as the commercial mice carrying the mutation in the rod-specific cyclic GMP phosphodiesterase gene, the trade name of which is Pdeb^{rd1/rd1}, from Jackson laboratories.

rd10 mice are understood as the commercial mice carrying the mutation in the rod-specific cyclic GMP phosphodiesterase gene, the trade name of which is Pdeb^{rd10/rd10}, from Jackson laboratories.

Proins/rd10^{-/-} mice are understood as the mice generated by crossing which are rd10 mice and transgenic human proinsulin-producing mice under the striated muscle promoter MLC1. The construct introduced in these mice carries the cDNA of human proinsulin protein controlled by light chain myosin muscular promoter, the expression of which is constitutive (Figure 1B, SEQ ID NO 1). A variability in the expression is observed, which can be due to the different penetrance of the transgenic mice. This is correlated with the production of human proinsulin found in serum.

The construct used to generate the transgenic mice consisted of a plasmid (pMLC-hIns) with a size of 6.4 kb. The expression is mediated by the myosin light chain constitutive muscular promoter (MLC). The cDNA of the human proinsulin gene is cloned between the two EcoRI sites (Figure 1B).
Genotyping. The genomic DNA was obtained from the tissues according to the technique described by Miller et al., 1988 (Miller, S.A., Dykes, D.D. and Polesky, H.F. (1988) A simple salting out procedure for extracting DNA from human nucleated cells. Nucleic Acids Res, 16, 1215.). The tails of weaned mice were digested in 0.5 ml of lysis buffer (40 mM Tris-HC1, pH 8.0, 20 mM EDTA, 0.5% SDS and 200 mM NaCl) with 0.3 mg of Proteinase K (Boche Diagnostics, Mannheim, Germany). Once the DNA was precipitated, it was cleaved with the HindIII enzyme (Roche). 10 µg of genomic DNA were used which were digested with the HindIII enzyme in a final volume of 50 µl, overnight at 37°C. They were loaded into each individual well of a 12 cm long 1% agarose gel for a good size separation. Prior to the transfer, the gel was prepared with the following solutions: first 15 minutes with depurination solution (0.5 M HCl), then 30 minutes with denaturing solution (0.5 N NaOH and 1.5 M NaCl), and finally 30 minutes with neutralizing solution (0.5 M Tris-HC1, pH 8). DNA fragmentation is achieved with this treatment, whereby its transfer is facilitated. Nylon membranes (Schleicher & Schuell BioScience, USA) were used and the DNA was fixed to the membrane by UV radiation in the Stratalinker oven (Stratagene, La Jolla, CA, USA). The wet transfer was carried out overnight at room temperature.

For the genotyping, a ³²P-radioactively labeled probe in the dCTP base against the human proinsulin cDNA sequence inserted in the construct was used. The template for making the probe was obtained from the plasmid itself, digesting with EcoRI (Roche).

The insert released after digesting the construct with EcoRI was purified by the DNA extraction kit (Millipore). The probe labeling was performed using the Random primer Kit (Stratagene) in the presence of [³²P]dCTP. The probe was subsequently purified in Microspin G25 columns (Amersham Pharmacia Biotech).

The membrane was prehybridized at 65°C with a solution containing 50% formamide, Denhardt 1x (0.02% Ficol, 0.02% polyvinylpyrrolodone and 0.02% BSA), 1% SDS, 5x SSC (0.15 M NaCl and 15 mM sodium citrate at pH 7.2) and 0.1 mg/ml of salmon sperm DNA for at least 2 hours; it was subsequently hybridized at 65°C overnight with the prehybridization solution, to which 1.5x10⁶ cpm/ml of the probe were added. Two washes of 15 minutes with 2x SSC at room temperature, another wash of 30 minutes with 2x SSC 1% SDS and a final wash of 30 minutes with 2x SSC and 0.1% SDS at 62.5°C were carried out.

Once the filters were washed, without allowing them to dry, they were wrapped in a GLAD type plastic and were exposed between two amplification screens (Genescreen plus, DuPont) to a Kodak Biomax MS type 18x24 cm photographic film (Eastman Kodak Company, Rochester, NY, USA). An exposure time of 3-4 days was required to obtain a sharp signal. The probe produced against human proinsulin cDNA detected a 1.5 Kb band in the genomic DNA gel.

rd10 mice have a point mutation located in exon 13 of the rod-specific cyclic GMP phosphodiesterase 6 enzyme gene. In wild-type mice there is a cleavage site with the CfoI enzyme in that location. In the case of mutants, the enzyme cleavage site disappears. This allowed an intrinsic genotyping technique control.

Genomic PCR was carried out with the following primers: 3-CTTTCTATTCTCTGTCAGCAAAGC-5 (Oligo A, SEQ ID NO 3) and 3-CATGAGTAGGGTAAACATGGTCTG-5 (Oligo B, SEQ ID NO 4) which amplified a 97 bp fragment. The PCR product was then subjected to digestion with the CfoI enzyme (Roche) for two hours at 37°C. It was then fractioned in a 3% Metaphor agarose gel. Wild-type mice showed two 54 and 43 bp bands, after digestion. Homozygous rd10 mice gave a single 97 bp band, because the enzyme did not cleave the amplicon and the heterozygous rd10/+ mice showed three bands (of an allele which is cleaved and the other one is not). No type of variability between individuals was found in this gene, which means that degeneration follows a standard pattern which is usually fulfilled in the same manner for all the individuals having it.

### 1.2.- Determination of blood sugar and of proinsulin levels in transgenic mice.

The blood sugar of all the mice was measured with test strips (Accu-Chek, Roche) after 12 hours of fasting. The normal levels of a mouse are usually between 100 and 200 mg/dl. A variation of between 80 and 150 mg/dl was found in Proins/rd10^{-/-} double mutants, which variation was not directly correlated with the proinsulinemia levels.

A commercial ELISA assay (Linco Research, MO, USA) was used to detect the human proinsulin production levels by transgenic mice, which specifically detects human proinsulin.

Muscle and serum of both transgenic and control mice were analyzed. In the case of serum, all the manufacturer's recommendations were followed but in the case of muscle, a prior protein extraction with a lysis buffer(50 mM Tris-HCl, pH 7, 0,100 mM NaCl and 0.1% Triton) and a quantification thereof with the BCS kit (Pierce, Rockford, IL, USA) were required.

Human proinsulin detected in muscle extracts was always very high and it had to be corrected by the amount of protein. Human proinsulin detected in serum ranged between 1 and 15 µM. This concentration was measured in a volume of 20 µl, according to the manufacturer's instructions. The measurements were made systematically at P30. This indicated that transgenic Proins/rd10^{-/-} mice produce human proinsulin in muscle and that it is poured into the blood circulation where it can reach the neural retina.

### 1.3.- Immunostaining in cryostat retinal sections of transgenic mice.

The retinal histology was analyzed in P32 mice (Figure 2), comparing wild-type mice which do not suffer from degeneration (A and D), Proins/rd10^{-/-} mice (B and E) and rd10^{-/-} mice (C and F), which do suffer from degeneration. On P32, at which degeneration is very advanced in the retinas of rd10^{-/-} mice, the number of photoreceptive cells per column, the condition and abundance of cones and the condition of the synapses was analyzed (Figure 3). It was thus intended to verify the condition of the retina in general and the progress of degeneration with different markers.

All the tissue was embedded Tissue-tec (Sakura Finetek Europe B.U. The Netherlands), they were frozen in dry ice and stored at -80°C until their processing. 12 µm thick cryostat sections were made. They were collected in poly-L-lysine coated slides (Fisher Biotech, Pittsburgh, USA) and kept at - 80°C until their use.

In all cases, whichever the staining to be carried out, after removing the slides from the freezer, they were left at room temperature for half an hour and were fixed with 4% paraformaldehyde (PFA) for 20 minutes, after which they were washed with PBS.

It was decided to label the remaining cones, because as they degenerate in a secondary manner, without having any mutation, they could give an idea of the condition of the retina and its maintenance. In turn, it was enough to count the number of rows of cells remaining in the thickness of the outer nuclear layer (ONL) to see the progress of degeneration. A fluorochrome Alexa 488-labeled agglutinin (Molecular Probes, Eugene, OR, USA) was used for 2 hours in a solution of PBS with 0.1% BSA for labeling the cones. The washes were carried out with a solution containing 1 mM MgCl₂ and 1 mM CaCl₂. The outer segments of the cones and the axon terminals thereof were thus detected. The sections were mounted with mounting medium with DAPI, to counterstain the nuclei.

The wild-type control mice without degeneration have between eight and twelve rows of nuclei in the ONL (Figure 2D). The number of cones, carrying the outer segments and also the synaptic buttons thereof in the OPL, is quite representative by the agglutinin staining observed in the OPL (Figure 2A). In the control rd10^{-/-} mouse, the number of rows of rods in the ONL was quite small, between 1 and 2 rows, because the degeneration at this point was quite high (Figure 2F). What is most surprising is that the cones had already started to degenerate at this point, although they were not primarily affected by the mutation (Figure 2C). It was observed that hardly any cone outer segments appeared and the number of synaptic buttons thereof was considerably reduced. In Proins/rd10^{-/-} mice, 5-6 rows of rod nuclei were observed in this case, which was one of the mice in which the greatest proinsulinemia was detected (Figure 2E). The condition of the cones was very good; in fact it was similar to wild-type mice. The outer segments thereof and the synaptic buttons in the OPL indicating the good cone conservation can be seen (Figure 2B). A variety in ONL conservation which was correlated with the blood proinsulin level was observed.

Furthermore, the condition of the plexiform layers (Figure 3), where the synaptic connections of the neurons take place, was analyzed, because this treatment with proinsulin intends not only to prevent or delay the death of degeneration but to keep the cells alive, either whether they have intrinsic damage or are altered in a secondary manner or whether they are functional. For the staining with SV2 (synaptic vesicle 2) antibody, the cryostat sections, after the fixing with 4% PFA, were permeated with 0.1% Triton x-100 and blocked with 10% NGS (normal goat serum) in PBS for 1 hour. The SV2 antibody, at a 1:50 dilution, is bound to a protein of the synaptic vesicles, thus labeling the retinal plexiform layers (outer plexiform layer, OPL, and the inner plexiform layer, IPL). It was incubated at 4°C overnight in the blocking solution. The incubation with the Alexa 488-conjugated secondary antibody (1/200) was carried out for 1 hour at room temperature. After the corresponding PBS washes, the sections were mounted with mounting medium with DAPI, to counterstain the nuclei.

Expression was observed in the two retinal plexiform layers, the outer plexiform layer (OPL) and the inner plexiform layer (IPL) (Figure 3). The inner plexiform layer, where the bipolar interneuron connections with the neurons projecting to the brain, the ganglion cells, occur, showed considerable staining and good condition in the three cases. The difference was in the outer plexiform layer, where the connections of the photoreceptive cells with the bipolar interneurons are mainly located. The OPL was quite defined and intense in the wild-type control mouse (Figure 3A) and in the transgenic Proins/rd10^{-/-} mouse (Figure 3B). The control rd10 mouse maintained the staining in the IPL, but the OPL conserved quite unorganized and little staining (Figure 3C). Even synapse projection attempts between the nuclear layers were found in this control rd10^{-/-} mouse due to the lack or organization and to the fact that the remaining neurons lose their synapses with the photoreceptors. This indicated that the functional condition of the retina in Proins/rd10^{-/-} mice is better than in rd10^{-/-} mice without treatment.

This showed that in the transgenic Proins/rd10^{-/-} mouse, human proinsulin can maintain synaptic connections longer than in the degeneration situation without treatment. This would involve that, in addition to delaying the degenerating cell death, it keeps them in good condition and they can carry out their biological function.

The histologically observed improvements in Proins/rd10^{-/-} mice over rd10^{-/-} mice are more obvious with the subsequent electroretinographic recordings (Figures 4 and 5).

In addition, to analyze the value of the proinsulin levels in the neuroprotective effect, assays were carried out with models other than the chronic and progressive damage that is found in genetic models of neurodegenerative diseases of the retina and of other parts of the nervous system, as well in those associated to senility. Thus, preliminary studies were conducted in which retinal ganglion cell death was caused in adult rats by means of acute damage - cutting of the optic nerve - and they were administered with human proinsulin subcutaneously. This treatment caused a slight delay in ganglion cell death, until the inevitable moment in this acute damage paradigm (data not shown). Human proinsulin was also administered by subcutaneous and intravitreal injection as a treatment in these rd mice (Figures 8 and 9), but the degeneration did not stop, although proinsulin injected subcutaneously was able to reach the neuroretina (Figure 7). These results indicate that serum proinsulin can reach damaged areas of the central nervous system and exert neuroprotective actions only when sustained and extended levels are reached. Example 2.- Human proinsulin improves the visual function assessed by ERG in transgenic Proins/rdl0^{-/-} mice compared to control and ill mice.

Although the mice were always kept in 12-hour light-darkness cycles, for the electroretinographic study, the mice were adapted to darkness overnight. For informative purposes, the cones are responsible for photopic (daytime) vision and the rods for scotopic (night) vision. The mice were anesthetized under a weak red light with an intraperitoneal injection of a solution containing ketamine (at 95 mg/kg) and xylazine (at 5 mg/kg). The pupils were dilated with a drop of a solution containing 1% tropicamide (Colircusi Tropicamida, Alcon Cusi, SA, El Masnou, Barcelona, Spain). The recording electrode is a lens which was placed in the mouse eye. The reference electrode was placed in the mouth and the ground electrode was placed on the tail. The anesthetized animals were placed in a Faraday cage and all the scotopic experiments were carried out in complete darkness. The electroretinographic responses induced by flashes of low intensity light produced by a Ganzfeld stimulator were thus recorded, allowing to record the responses generated in rods only (rod response) or cones and rods (mixed response) in adaptation to darkness. The intensity of the light stimuli used were set to values comprised between -4 and 1.52 log cd.s.m⁻². The light intensity was determined by means of a photometer (Mavo Monitor USB) at the eye level. A maximum of 64 responses was averaged for each stimulus intensity. The interval between light stimuli changes depending on the intensity, thus, for low intensity stimuli (-4 log cd.s.m⁻²) the time between stimuli was set to 10 seconds and for high intensity stimuli (1.52 log cd.s.m⁻²) it was 60 seconds. The animal was adapted to photopic conditions for the purpose of recording cone isolated responses. Under these conditions, the interval between light flashes was set to 1 second.

The electric signals from the retina were amplified and filtered between 0.3 and 1000 Hz with a Grass amplifier (CP511 AC amplifier, Grass Instruments, Quincy, MA). The signals were digitized (PC-card ADI instruments, CA). The recordings were stored in the computer for their subsequent analysis.

The rod-mediated responses were recorded in darkness adaptation conditions, before the application of light flashes with intensities comprised between -4 and -1.52 log cd.s.m⁻². The mixed responses generated by the cones and rods were recorded before the application of light flashes with intensities comprised between -1.52 and 0.48 log cd.s.m⁻². The oscillating potentials were also isolated by means of applying electric filters comprised between 100 and 1000 Hz. The cone-mediated response was recorded in light adaptation conditions (recording background light of 30 cd.m⁻²), before the application of light flashes with intensities comprised between -0.52 and 2log cd.s.m⁻². The Flicker responses (30 Hz) were recorded in adaptation to light, before stimuli of 1.48 log cd.s.m⁻².

Figure 4 shows the electroretinographic responses recorded both in adaptation to darkness (scotopic conditions) and in adaptation to light (photopic conditions) of wild-type (WT), control rd10^{-/-} and Proins/rd10^{-/-} mice obtained at P30, both from line 1 and from line 2.

Figure 5 shows the result of analyzing the correlation between the electroretinographic responses between insulin and the proinsulin levels in a larger group or mice. This correlation suggests a dose-response relationship supporting the possible efficiency of a pharmacological approach with human proinsulin.

In addition, Figure 6 shows the electroretinographic records corresponding to wild-type (wt), control rd10 and Proins/rd10^{-/-} mice from line 2 obtained at different times of postnatal development (P35, P45 and P55). The recordings obtained in scotopic (adaptation to darkness) and photopic (adaptation to light) conditions are shown separately. It was observed how wild-type mice generated scotopic (rods) and photopic (cones) electroretinographic responses with a wide range at P35. The responses were nil at P35 in control rd10^{-/-} mice, both the responses generated in rods and in cones. Proins/rd10^{-/-} mice maintained very significant photopic and scotopic electroretinographic responses at P35 and achieved maintaining a certain degree of response up to P55. It was thus observed that the visual response was better in transgenic Proins/rd10^{-/-} mice and is more extended over time.

### Example 3. -Effect of proinsulin by intravitreal injection into the retina of rd1 mice.

To carry out the following Examples 3 and 4, rd1 type mice, sharing the same C57BL/6 genetic background with rd10 mice, as well as a different mutation but in the same rod-specific cyclic GMP phosphodiesterase gene, were used.

With regard to the effect of proinsulin by intravitreal injection into the retina of rd1 mice *in vivo,* specifically the intravitreal injection of 1 µg, at a concentration of 1 µg/µl, of human proinsulin in rd1 mice at P13. The right eyes were injected with proinsulin and the left eyes with the carrier. The injections were single injections and the effects were analyzed 24 or 48 hours later by TUNEL, both in retinas mounted in a planar manner and in sections. In the latter, the number of photoreceptors remaining in the outer nuclear layer was also evaluated. The TUNEL (TdT-mediated dUTP Nick End Labeling) technique was used to detect cell death. The terminal transferase enzyme (TdT) adds fluorescein-labeled nucleotides (dUTP) to the free 3'OH ends of the DNA, which allows detecting the DNA fragmentation occurring during programmed cell death. The Promega TUNEL kit was used. The technique was carried out on cells, sections or tissues. After a permeabilization step according to the nature of the tissue, it was washed with PBS and preincubated with the kit solution for 30 minutes at room temperature. The reaction mixture was prepared according to the manufacturer's instructions and the reaction was carried out for 1 hour at 37°C. The reaction was then stopped with 2x SSC solution for 15 minutes at room temperature. It was washed with PBS and mounted with Vectashield. To detect cell death in retinas mounted in a planar manner, the whole neural retina, dissected from the remaining elements of the eye, was mounted under the microscope on a black nitrocellulose membrane (Sartorius, Goettingen, Germany), for better contrast during handling. It was generally placed with the photoreceptor layer placed upwards, being adhered to the nitrocellulose with the aid of fine dissecting forceps. The retinas mounted in a planar manner were fixed with 4% (w/v) PFA in 0.1 M phosphate buffer, pH 7.1, overnight at 4°C in 24-well plates. On the next day, they were washed with PBS and with BSA (30 mg/ml in PBS). The permeabilization was carried out with 1% (w/v) Triton X-100 in PBS (4 times, 30 minutes each time) and enzymatically, with collagenase and proteinase K, previously eliminating the Triton X-100 residues by washing well with PBS. The collagenase (20 U/ml) was allowed to act for 1 hour at 37°C and then proteinase K (20 µg/ml) for 15 minutes at 37°C. It was then necessary to refix the retinas for at least two hours. They were washed well with PBS and with BSA (30 mg/ml in PBS) and the TUNEL reaction was carried out as indicated above. The retinas mounted in a planar manner were analyzed by confocal microscopy (Leica TCS-SP2-AOBS).

To locate cell death within the retinal layers, the TUNEL technique was carried out on retinal sections such as those described above, always fixed in 4& (w/v) PFA in 0.1 M phosphate buffer, pH 7.1 and cryoprotected with 30% (w/v) sucrose in 10 mM phosphate buffer, pH 7.1. For the TUNEL labeling in sections, less permeation is required than of retinas mounted in a planar manner. Thus, two series of permeation with BGT [100 mM glycine, 3 mg/ml BSA, 0.25% (w/v) Triton X-100 in PBS] of 15 minutes each were carried out. They were washed with PBS and the TUNEL reaction was carried out as indicated. To cover all the sections of a slide, 50 µl of the corresponding reagents were added and they were covered with parafilm®.

None of the quantifications (TUNEL in sections, TUNEL in retinas mounted in a planar manner, photoreceptors in sections) provided significant differences between the treatment with proinsulin and the carrier.

### Example 4.- Effect of proinsulin by subcutaneous injections in rd1 mice.

A new route of administration, subcutaneous injection, was tested in order to carry out more extended treatments. This route, routinely used for insulin administration in diabetic patients, is much less traumatic than intravitreal injection. Furthermore, the human proinsulin administration in this manner had given preliminary positive delay results in retinal ganglion cell degeneration after cutting the optic nerve in adult rats. A number of protocols were carried out to optimize the pharmacological supply of human proinsulin (Table).

**TABLE: It shows the experimental approach followed in each of the attempts of subcutaneous injection of human proinsulin. In all cases, the injected amount was 1.6 µg of human proinsulin, except in case 1, which was a single dose of 5 µg and in case 5, from P14 to P18, which was of 2.5 µg. (nd= not determined). The number of animals relates to the total of siblings per litter and experiment, half of them normally being injected with PBS and the other half with human proinsulin.**

| INTERVAL | Injection Frequency | Number of mice | Rod maintenance | Visual function | Presence in retina |
|---|---|---|---|---|---|
| P14 (5 µg) | single | 6 | nd | nd | + (Fig. 7) |
| P11-P14 | 24h | 6 | nd | nd | + (Fig. 7) |
| P8-P15 | 24h | 6 | - | nd | nd |
| P6-P14 | 24h | 6 | - | nd | nd |
| P4-P14-P18 | 12h | 7 | - (Fig. 8) | -(Fig. 9) | nd |

It was first determined if proinsulin reached the retina by means of subcutaneous injection. To that end, studies were conducted in mice injected subcutaneously with proinsulin by means of an ELISA that detected human proinsulin (Figure 7) (Linco Research, MO, USA).

This assay was designed to detect serum proinsulin, so it had to be adapted to muscle and retinal extracts. To detect the human proinsulin production levels by transgenic mice, muscle and retinal, as well as serum, extracts were analyzed. In the case of serum, blood was drawn from the lacrimal sac area of mice eyes with a Pasteur pipette. It was transferred to an eppendorf tube where it was allowed to clot for 2 hours at room temperature and was centrifuged at 1,300 g for 15 minutes, to collect the serum, which was frozen until its use. In the case of the tissues, a protein extraction was carried out with a lysis buffer [50 mM Tris-HCl, pH 7, 100 mM NaCl and 0.1% (w/v) Triton X-100] and a quantification of the extracted protein with the BCA kit (Pierce) were carried out. The neural retinas were extracted with a volume of 60 µl each. The muscles of the hind legs were extracted in a volume of between 200 and 300 µl, according to the piece of muscle obtained.

In the case of serum, all the manufacturer's recommendations were followed, always putting 20 µl duplicates. In the case of the tissues, 20 µl of extract were also placed in duplicate, serial dilutions being occasionally used for greater accuracy. The retinal and muscle extract data were corrected by the amount of protein.

It was possible to identify human proinsulin in a P14 wild-type mouse retinal extract only 2 hours after a subcutaneous injection, in a dose-dependent manner. A 1.6 µg dose produced a retinal concentration of 0.72 pM, whereas a 5 µg dose produced 3.62 pM. The daily administration of 1.6 µg between P11 and P14 was able to produce higher, possibly accumulated, levels of 3.56 pM. Thus, this approach confirmed the capacity of peripheral exogenous proinsulin to reach the neural retina and the greater effectiveness of a repetitive treatment, at least in achieving higher proinsulin levels in the retina.

The effect of repetitive treatment in various intervals on photoreceptors in neurodegeneration and on the visual function determined by electroretinogram (Table; Figures 8 and 9) was verified. A daily dose of 1.6 µg between P8 and P15 was first injected. In this case, there was no protective effect at the histological level. To start the rescue earlier, with the belief that the effect could be more evident if proinsulin was administered before the degenerative process started, the treatment was tested between P6 and P14, the maintenance of the photoreceptors not being obtained this time. For the purpose of attempting histological recovery and also of evaluating the visual function by means of electroretinograms, the protocol was modified, two daily injections of 1.6 µg of human proinsulin between P6 and P14 being carried out. Some animals were maintained up to P18, the proinsulin dose being increased to 2.5 µg in this second period. This treatment had no protective effects at the histological level (Figure 8) or at the functional level (Figure 9).

The most usual result was lack of histological recovery and lack of visual function by ERG (Figure 8 and Figure 9), despite the fact that injected proinsulin was able to reach the retina in which all the elements enabling a response to it were apparently located.

## Claims

1. The use of a compound that induces the activity of proinsulin wherein said compound is selected from the group consisting of
a.a nucleotide sequence allowing the expression of a neuroprotective protein or peptide and which is formed by one or several nucleotide sequences belonging to the group of
i. a nucleotide sequence formed by the nucleotide sequence encoding human proinsulin SEQ ID NO 1,
ii. a nucleotide sequence which has a degree of identity of at least 95% to the sequence of i)and encoding a protein mimicking the activity of human proinsulin and,
iii. a nucleotide sequence comprising any one sequence belonging to i) and/or ii);
b.an isolated human eukaryotic cell which is genetically modified and transformed by means of the human proinsulin nucleotide sequence SEQ ID NO:1 and comprises the construct or expression vector and can suitably release the proinsulin protein to the extracellular medium and
c.a protein or peptide having neuroprotective activity and comprising one or several amino acid sequences belonging to the group of
i. an amino acid sequence formed by the human proinsulin amino acid sequence SEQ ID NO: 2,
ii. an amino acid sequence which has a degree of identity of at least 95% to the sequence of i) and mimics the neuroprotective activity of human proinsulin and,
iii. an amino acid sequence comprising any one sequence belonging to: i) and/or ii)
for the preparation of a medicinal product or pharmaceutical composition for the prevention and treatment of retinitis pigmentosa.

2. The use of a compound according to claim 1 wherein the compound is to be administered in a sustained manner so as to obtain chronic proinsulinemia levels of 1-15 pM.

3. The use of a compound according to claim 1 wherein the compound is to be administered intravitreally.

4. The use according to claim 1, **characterized in that** the nucleotide sequence is formed by SEQ ID NO 1 encoding human proinsulin.

5. The use according to claim 1, **characterized in that** the nucleotide sequence is formed by a gene construct comprising the proinsulin nucleotide sequence SEQ ID NO 1.

6. The use according to claim 1, **characterized in that** the nucleotide sequence is formed by an expression vector comprising a nucleotide sequence or a genetic construct encoding a proinsulin protein which can induce neuroprotection.

7. The use according to claim 6, **characterized in that** the expression vector contains the nucleotide sequence SEQ ID NO 1 and a tissue-specific, preferably a muscle-specific promoter.

8. The use according to claim 1, **characterized in that** the inducer compound is the human proinsulin protein SEQ ID NO 2.

9. A pharmaceutical composition or medicinal product for use in the treatment of retinitis pigmentosa, **characterized in that** it comprises a compound that induces the activity of proinsulin in a therapeutically effective amount together with one or more pharmaceutically acceptable adjuvants and/or carriers wherein the compound is selected from the group of
a.a nucleotide sequence allowing the expression of a neuroprotective protein or peptide and which is formed by one or several nucleotide sequences belonging to the group of
i. a nucleotide sequence formed by the nucleotide sequence encoding human proinsulin SEQ ID NO 1,
ii. a nucleotide sequence which has a degree of identity of at least 95% to the sequence of i) and encoding a protein mimicking the activity of human proinsulin and,
iii. a nucleotide sequence comprising any one sequence belonging to i) and/or ii);
b.an isolated human eukaryotic cell which is genetically modified and transformed by means of the human proinsulin nucleotide sequence SEQ ID NO 1 and comprises the construct or expression vector and can suitably release the proinsulin protein to the extracellular medium;
c.a protein or peptide having neuroprotective activity and comprising one or several amino acid sequences belonging to the group of
i. an amino acid sequence formed by the human proinsulin amino acid sequence SEQ ID NO 2,
ii. an amino acid sequence which has a degree of identity of at least 95% to the sequence of i), and mimics the neuroprotective activity of human proinsulin and,
iii. an amino acid sequence comprising any one sequence belonging to: i) and/or ii)

10. A pharmaceutical composition for use in the treatment of retinitis pigmentosa according to claim 9 suitable for maintaining a systemic or local sustained administration of the inducer compound so as to obtain chronic proinsulinemia levels of 1-15 pM.

11. A pharmaceutical composition for use in the treatment of retinitis pigmentosa according to claim 9 wherein the composition is to be administered intravitreally.

12. A pharmaceutical composition for use in the treatment of retinitis pigmentosa according to claim 9, **characterized in that** the nucleotide sequence is formed by the SEQ ID NO 1 encoding human proinsulin.

13. A pharmaceutical composition for use in the treatment of retinitis pigmentosa according to claim 9, **characterized in that** the nucleotide sequence of claim 9 a iii) is formed by a gene construct comprising the proinsulin nucleotide sequence SEQ ID NO 1.

14. A pharmaceutical composition for use in the treatment of retinitis pigmentosa according to claim 9, **characterized in that** the nucleotide sequence of claim 9 a iii) is an expression vector comprising a nucleotide sequence or a gene construct encoding a proinsulin protein which can induce neuroprotection.

15. A pharmaceutical composition for use in the treatment of retinitis pigmentosa according to claim 14, **characterized in that** the expression vector contains the nucleotide sequence SEQ ID NO 1 and a tissue-specific, preferably a muscle-specific promoter.

16. A pharmaceutical composition for use in the treatment of retinitis pigmentosa according to claim 9, **characterized in that** the amino acid sequence is formed by human proinsulin SEQ ID NO 2.

17. A pharmaceutical composition for use in the treatment of retinitis pigmentosa according to claim 9, **characterized in that** the isolated human eukayotic cell which is genetically modified and transformed by means of the human proinsulin nucleotide sequence of SEQ ID NO: 1 and comprises the construct or expression vector and can suitable release the proinsulin protein to the extracellular medium, is a central nervous system cell.

## Patentansprüche

1. Verwendung einer Verbindung, die die Aktivität von Proinsulin induziert, worin die Verbindung aus der Gruppe ausgewählt ist, die aus folgendem besteht
a. einer Nukleotidsequenz, die die Expression eines neuroprotektiven Proteins oder Peptids ermöglicht, und die aus einer oder mehrerer Nukleotidsequenzen gebildet wird, die zur folgenden Gruppe gehören:
i. eine Nukleotidsequenz, die aus der Nukleotidsequenz gebildet wird, die humanes Proinsulin SEQ ID NO:1 kodiert,
ii. eine Nukleotidsequenz, die einen Identitätsgrad von mindestens 95% zur Sequenz von i) aufweist und ein Protein kodiert, das die Aktivität von humanem Proinsulin imitiert und
iii. eine Nukleotidsequenz, die irgendeine Sequenz umfasst, die zu i) und/oder ii) gehört;
b. eine isolierte, humane, eukariotische Zelle, die genetisch modifiziert und mit der humanen Proinsulinnukleotidsequenz SEQ ID NO:1 transformiert ist und das Konstrukt oder den Expressionsvektor umfasst und das Proinsulinprotein geeigneterweise ins extrazelluläre Medium freisetzen kann und
c. ein Protein oder Peptid, das neuroprotektive Aktivität aufweist und eine oder mehrere Aminosäuresequenzen umfasst, die zur folgenden Gruppe gehören
i. eine Aminosäuresequenz, die von der humanen Proinsulinaminosäuresequenz SEQ ID NO:2 gebildet wird,
ii. eine Aminosäuresequenz, die einen Identitätsgrad von mindestens 95% zur Sequenz aus i) aufweist und die neuroprotektive Aktivität von humanem Proinsulin imitiert, und
iii. eine Aminosäuresequenz, die irgendeine Sequenz umfasst, die zu i) und/oder ii) gehört zur Herstellung eines medizinischen Erzeugnisses oder einer pharmazeutischen Zusammensetzung zur Prävention und Behandlung von Retinitis pigmentosa.

2. Verwendung einer Verbindung gemäß Anspruch 1, worin die Verbindung in fortwährender Weise zu verabreichen ist, um chronische Proinsulinspiegel von 1-15 pM zu erreichen.

3. Verwendung einer Verbindung gemäß Anspruch 1, worin die Verbindung intravitreal zu verabreichen ist.

4. Verwendung gemäß Anspruch 1, **dadurch gekennzeichnet, dass** die Nukleotidsequenz durch SEQ ID NO:1 gebildet wird, die humanes Proinsulin kodiert.

5. Verwendung gemäß Anspruch 1, **dadurch gekennzeichnet, dass** die Nukleotidsequenz durch ein Genkonstrukt gebildet wird, das die Proinsulin-Nukleotidsequenz SEQ ID NO:1 umfasst.

6. Verwendung gemäß Anspruch 1, **dadurch gekennzeichnet, dass** die Nukleotidsequenz durch einen Expressionsvektor gebildet wird, der eine Nukleotidsequenz oder ein Genkonstrukt umfasst, die ein Proinsulinprotein kodiert, das Neuroprotektion induzieren kann.

7. Verwendung gemäß Anspruch 6, **dadurch gekennzeichnet, dass** der Expressionsvektor die Nukleotidsequenz SEQ ID NO:1 und einen gewebespezifischen, vorzugsweise einen muskelspezifischen Promotor umfasst.

8. Verwendung gemäß Anspruch 1, **dadurch gekennzeichnet, dass** die Induktorverbindung das humane Proinsulinprotein SEQ ID NO:2 ist.

9. Pharmazeutische Zusammensetzung oder medizinisches Erzeugnis zur Verwendung in der Behandlung von Retinitis pigmentosa, **dadurch gekennzeichnet, dass** sie/es eine Verbindung, die die Aktivität von Proinsulin induziert, in einer therapeutisch wirksamen Menge zusammen mit einem oder mehreren pharmazeutisch annehmbaren Hilfsstoffen und/oder Trägern umfasst, worin die Verbindung aus der Gruppe ausgewählt ist, die aus folgendem besteht:
a. einer Nukleotidsequenz, die die Expression eines neuroprotektiven Proteins oder Peptids ermöglicht, und die aus einer oder mehrerer Nukleotidsequenzen gebildet wird, die zur folgenden Gruppe gehören:
i. eine Nukleotidsequenz, die aus der Nukleotidsequenz gebildet wird, die humanes Proinsulin SEQ ID NO:1 kodiert,
ii. eine Nukleotidsequenz, die einen Identitätsgrad von mindestens 95% zur Sequenz von i) aufweist und ein Protein kodiert, das die Aktivität von humanem Proinsulin imitiert und
iii. einer Nukleotidsequenz, die irgendeine Sequenz umfasst, die zu i) und/oder ii) gehört;
b. eine isolierte, humane, eukariotische Zelle, die genetisch modifiziert und mit der humanen Proinsulin-Nukleotidsequenz SEQ ID NO:1 transformiert ist und das Konstrukt oder den Expressionsvektor umfasst und das Proinsulinprotein geeigneterweise ins extrazelluläre Medium freisetzen kann und
c. ein Protein oder Peptid, das neuroprotektive Aktivität aufweist und eine oder mehrere Aminosäuresequenzen umfasst, die zur folgenden Gruppe gehören:
i. eine Aminosäuresequenz, die von der humanen Proinsulinaminosäuresequenz SEQ ID NO:2 gebildet wird,
ii. eine Aminosäuresequenz, die einen Identitätsgrad von mindestens 95% zur Sequenz aus i) aufweist und die neuroprotektive Aktivität von humanem Proinsulin imitiert, und
iii. eine Aminosäuresequenz, die irgendeine Sequenz umfasst, die zu i) und/oder ii) gehört.

10. Pharmazeutische Zusammensetzung zur Verwendung in der Behandlung von Retinitis pigmentosa gemäß Anspruch 9, die geeignet ist, eine systemisch oder lokal fortwährende Verabreichung der Induktorverbindung aufrecht zu erhalten, um einen chronischen Proinsulinspiegel von 1-15 pM zu erreichen.

11. Pharmazeutische Zusammensetzung zur Verwendung in der Behandlung von Retinitis pigmentosa gemäß Anspruch 9, worin die Zusammensetzung intravitreal zu verabreichen ist.

12. Pharmazeutische Zusammensetzung zur Verwendung in der Behandlung von Retinitis pigmentosa gemäß Anspruch 9, **dadurch gekennzeichnet, dass** die Nukleotidsequenz durch die SEQ ID NO:1 gebildet wird, die humanes Proinsulin kodiert.

13. Pharmazeutische Zusammensetzung zur Verwendung in der Behandlung von Retinitis pigmentosa gemäß Anspruch 9, **dadurch gekennzeichnet, dass** die Nukleotidsequenz von Anspruch 9 a.iii) aus einem Genkonstrukt gebildet wird, das die Proinsulin-Nukleotidsequenz SEQ ID NO:1 umfasst.

14. Pharmazeutische Zusammensetzung zur Verwendung in der Behandlung von Retinitis pigmentosa gemäß Anspruch 9, **dadurch gekennzeichnet, dass** die Nukleotidsequenz von Anspruch 9 a.iii) ein Expressionsvektor ist, der eine Nukleotidsequenz oder ein Genkonstrukt umfasst, die/das ein Proinsulinprotein kodiert, das Neuroprotektion induzieren kann.

15. Pharmazeutische Zusammensetzung zur Verwendung in der Behandlung von Retinitis pigmentosa gemäß Anspruch 14, **dadurch gekennzeichnet, dass** der Expressionsvektor die Nukleotidsequenz SEQ ID NO:1 und einen gewebespezifischen, vorzugsweise einen muskelspezifischen Promotor umfasst.

16. Pharmazeutische Zusammensetzung zur Verwendung in der Behandlung von Retinitis pigmentosa gemäß Anspruch 9, **dadurch gekennzeichnet, dass** die Aminosäuresequenz durch humanes Proinsulin SEQ ID NO:2 gebildet wird.

17. Pharmazeutische Zusammensetzung zur Verwendung in der Behandlung von Retinitis pigmentosa gemäß Anspruch 9, **dadurch gekennzeichnet, dass** die isolierte, humane, eukariotische Zelle, die genetisch modifiziert und mit der humanen Proinsulinnukleotidsequenz SEQ ID NO:1 transformiert ist und das Konstrukt oder den Expressionsvektor umfasst und das Proinsulinprotein geeigneterweise ins extrazelluläre Medium freisetzen kann, eine Zelle des Zentralnervensystems ist.

## Revendications

1. Utilisation d'un composé qui induit l'activité de la proinsuline, dans laquelle ledit composé est sélectionné dans le groupe constitué par
a. une séquence nucléotidique permettant l'expression d'une protéine neuroprotectrice ou d'un peptide neuroprotecteur et qui est formée par une ou plusieurs séquences nucléotidiques appartenant au groupe constitué par
i. une séquence nucléotidique formée par la séquence nucléotidique codant pour la proinsuline humaine SEQ ID NO : 1,
ii. une séquence nucléotidique qui a un degré d'identité d'au moins 95 % avec la séquence de i) et codant pour une protéine imitant l'activité de la proinsuline humaine et,
iii. une séquence nucléotidique comprenant l'une quelconque des séquences appartenant à i) et/ou ii) ;
b.une cellule eucaryote humaine isolée qui est génétiquement modifiée et transformée au moyen de la séquence nucléotidique de proinsuline humaine SEQ ID NO : 1 et comprend la construction génique ou le vecteur d'expression et peut libérer de manière appropriée la protéine de proinsuline dans le milieu extracellulaire et
c.une protéine ou un peptide ayant une activité neuroprotectrice et comprenant une ou plusieurs séquences d'acides aminés appartenant au groupe constitué par
i. une séquence d'acides aminés formée par la séquence d'acides aminés de proinsuline humaine SEQ ID NO : 2,
ii. une séquence d'acides aminés qui a un degré d'identité d'au moins 95 % avec la séquence de i) et imite l'activité neuroprotectrice de la proinsuline humaine et,
iii. une séquence d'acides aminés comprenant l'une quelconque des séquences appartenant à : i) et/ou ii)
pour la préparation d'un produit médicinal ou d'une composition pharmaceutique pour la prévention et le traitement de la rétinite pigmentaire.

2. Utilisation d'un composé selon la revendication 1, dans laquelle le composé est destiné à être administré d'une manière prolongée de manière à obtenir des niveaux de proinsulinémie chronique de 1 à 15 pM.

3. Utilisation d'un composé selon la revendication 1, dans laquelle le composé est destiné à être administré par voie intravitréale.

4. Utilisation selon la revendication 1, **caractérisée en ce que** la séquence nucléotidique est formée par la séquence SEQ ID NO : 1 codant pour la proinsuline humaine.

5. Utilisation selon la revendication 1, **caractérisée en ce que** la séquence nucléotidique est formée par une construction génique comprenant la séquence nucléotidique de proinsuline SEQ ID NO : 1.

6. Utilisation selon la revendication 1, **caractérisée en ce que** la séquence nucléotidique est formée par un vecteur d'expression comprenant une séquence nucléotidique ou une construction génique codant pour une protéine de proinsuline qui peut induire une neuroprotection.

7. Utilisation selon la revendication 6, **caractérisée en ce que** le vecteur d'expression contient la séquence nucléotidique SEQ ID NO : 1 et un promoteur tissu-spécifique, de préférence muscle-spécifique.

8. Utilisation selon la revendication 1, **caractérisée en ce que** le composé inducteur est la protéine de proinsuline humaine SEQ ID NO : 2.

9. Composition pharmaceutique ou produit médicinal destiné à être utilisé dans le traitement de la rétinite pigmentaire, **caractérisé en ce qu'**il comprend un composé qui induit l'activité de la proinsuline en une quantité thérapeutiquement efficace conjointement avec un ou plusieurs adjuvants et/ou véhicules pharmaceutiquement acceptables, le composé étant sélectionné dans le groupe constitué par
a. une séquence nucléotidique permettant l'expression d'une protéine neuroprotectrice ou d'un peptide neuroprotecteur et qui est formée par une ou plusieurs séquences nucléotidiques appartenant au groupe constitué par
i. une séquence nucléotidique formée par la séquence nucléotidique codant pour la proinsuline humaine SEQ ID NO : 1,
ii. une séquence nucléotidique qui a un degré d'identité d'au moins 95 % avec la séquence de i) et codant pour une protéine imitant l'activité de la proinsuline humaine et,
iii. une séquence nucléotidique comprenant l'une quelconque des séquences appartenant à i) et/ou ii) ;
b.une cellule eucaryote humaine isolée qui est génétiquement modifiée et transformée au moyen de la séquence nucléotidique de proinsuline humaine SEQ ID NO : 1 et comprend la construction génique ou le vecteur d'expression et peut libérer de manière appropriée la protéine de proinsuline dans le milieu extracellulaire ;
c.une protéine ou un peptide ayant une activité neuroprotectrice et comprenant une ou plusieurs séquences d'acides aminés appartenant au groupe constitué par
i. une séquence d'acides aminés formée par la séquence d'acides aminés de proinsuline humaine SEQ ID NO : 2,
ii. une séquence d'acides aminés qui a un degré d'identité d'au moins 95 % avec la séquence de i) et imite l'activité neuroprotectrice de la proinsuline humaine et,
iii. une séquence d'acides aminés comprenant l'une quelconque des séquences appartenant à : i) et/ou ii).

10. Composition pharmaceutique destinée à être utilisée dans le traitement de la rétinite pigmentaire selon la revendication 9 appropriée pour maintenir une administration prolongée systémique ou locale du composé inducteur de manière à obtenir des niveaux de proinsulinémie chronique de 1 à 15 pM.

11. Composition pharmaceutique destinée à être utilisée dans le traitement de la rétinite pigmentaire selon la revendication 9, dans laquelle la composition est destinée à être administrée par voie intravitréale.

12. Composition pharmaceutique destinée à être utilisée dans le traitement de la rétinite pigmentaire selon la revendication 9, **caractérisée en ce que** la séquence nucléotidique est formée par la séquence SEQ ID NO : 1 codant pour la proinsuline humaine.

13. Composition pharmaceutique destinée à être utilisée dans le traitement de la rétinite pigmentaire selon la revendication 9, **caractérisée en ce que** la séquence nucléotidique de la revendication 9 a iii) est formée par une construction génique comprenant la séquence nucléotidique de proinsuline SEQ ID NO : 1.

14. Composition pharmaceutique destinée à être utilisée dans le traitement de la rétinite pigmentaire selon la revendication 9, **caractérisée en ce que** la séquence nucléotidique de la revendication 9 a iii) est un vecteur d'expression comprenant une séquence nucléotidique ou une construction génique codant pour une protéine de proinsuline qui peut induire une neuroprotection.

15. Composition pharmaceutique destinée à être utilisée dans le traitement de la rétinite pigmentaire selon la revendication 14, **caractérisée en ce que** le vecteur d'expression contient la séquence nucléotidique SEQ ID NO : 1 et un promoteur tissu-spécifique, de préférence muscle-spécifique.

16. Composition pharmaceutique destinée à être utilisée dans le traitement de la rétinite pigmentaire selon la revendication 9, **caractérisée en ce que** la séquence d'acides aminés est formée par la séquence SEQ ID NO : 2 de proinsuline humaine.

17. Composition pharmaceutique destinée à être utilisée dans le traitement de la rétinite pigmentaire selon la revendication 9, **caractérisée en ce que** la cellule eucaryote humaine isolée qui est génétiquement modifiée et transformée au moyen de la séquence nucléotidique de proinsuline humaine de SEQ ID NO : 1 et comprend la construction génique ou le vecteur d'expression et peut libérer de manière appropriée la protéine de proinsuline dans le milieu extracellulaire est une cellule du système nerveux central.


### SEQUENCE LISTING

<110> Consejo superior de investigaciones Científicas Universidad de Alcalá de Henares Universitat Autònoma de Barcelona
<120> Use of proinsulin for the preparation of a neuroprotective pharmaceutical composition, therapeutic composition containing it and application thereof
<130> P3580EPPC
<140> EP07765882.1
   <141> 2007-05-21
<150> ES P200601314
   <151> 2006-05-22
<160> 4
<170> PatentIn version 3.5
<210> 1
   <211> 1125
   <212> DNA
   <213> Homo sapiens
<400> 1
   actttctttt tgaggttctt ctggaggaga tccttctttt gttccaagct atcgaattct      60
   gccatggccc tgtggatgcg cctcctgccc ctgctggcgc tgctggccct ctggggacct     120
   gacccagccg cagcctttgt gaaccaacac ctgtgcggct cacacctggt ggaagctctc     180
   tacctagtgt gcggggaacg aggcttcttc tacacaccca agacccgccg ggaggcagag     240
   gacctgcagg tggggcaggt ggagctgggc gggggccctg gtgcaggcag cctgcagccc     300
   ttggccctgg aggggtccct gcagaagcgt ggcattgtgg aacaatgctg taccagcatc     360
   tgctccctct accagctgga gaactactgc aactagacgc agctgcaagc ttatcgatac     420
   cgtccggaat tcctgcagcc cgggggatct ttgtgaagga accttacttc tgtggtgtga     480
   cataattgga caaactacct acagagattt aaagctctaa ggtaaatata aaatttttaa     540
   gtgtataatg tgttaaacta ctgattctaa ttgtttgtgt attttagatt ccaacctatg     600
   ggaactgatg aatgggagca gtggtggaat ggctttaatg aggaaaacct gttttgctca     660
   gaagaaatgc catctagtga tgatgaaggc tactgctgac tctcaacatt ctactcctcc     720
   aaaaaagaag agaaacggta gaaggaccgc tcgactttcc ttcagaattg ctaaggtttt     780
   tgagtcatgc tgtgattagt aatagaactg cttgccttgc atttgctatt tacacccaca     840
   aaggaaaaag cctgcactgc tatacaagaa aaattattgg aaaaattatt cttgtaacct     900
   ttatagtagg cataaacagt tattaatcat aacatacctg gttgttttgc tttacttcca     960
   caccaggcat agaggggatc tgcttattta tgtactattg ctgaaatgtg tgtaccgtta    1020
   gcttttttta ttttgttaag cgtcataaag aaaatattcg aggttaatgg cttggcttaa    1080
   gattcttatt cgccattcca atttgaaggg ttactggcgt tggac                    1125
<210> 2
   <211> 110
   <212> PRT
   <213> Homo sapiens
<400> 2
<210> 3
   <211> 24
   <212> DNA
   <213> Artificial Sequence
<220>
   <223> Oligo A
<400> 3
   ctttctattc tctgtcagca aagc 24
<210> 4
   <211> 24
   <212> DNA
   <213> Artificial sequence
<220>
   <223> Oligo B
<400> 4
   catgagtagg gtaaacatgg tctg 24